# EUROPEAN PATENT SPECIFICATION

(11) **EP 3 306 303 B1**
(45) Date of publication and mention of the grant of the patent: **10.07.2024**
(21) Application number: 16802944.5
(22) Date of filing: 20.04.2016
(51) Int. Cl.: G01N 33/00, G01N 21/27

(54) **WAVELENGTH SENSOR DEVICE FOR PLANT**
WELLENLÄNGENSENSORVORRICHTUNG FÜR PFLANZEN
DISPOSITIF DE DÉTECTION DE LONGUEUR D'ONDE POUR PLANTE

(30) Priority: 02.06.2015 JP 2015112242
(43) Date of publication of application: 11.04.2018
(73) Proprietor: Topcon Corporation, Tokyo 174-8580 (JP)
(72) Inventor: AKIYAMA, Shugo, Tokyo 174-8580 (JP); ZHAO, Peng, Tokyo 174-8580 (JP); HANYA, Issei, Tokyo 174-8580 (JP)
(74) Representative: Pfenning, Meinig & Partner mbB
(86) International application number: PCT/JP2016/062565
(87) International publication number: WO 2016/194507

(56) References cited:
- JP-A- 2008 175 537
- JP-A- 2010 054 436
- JP-A- 2012 183 021
- JP-A- 2013 000 090
- JP-A- 2013 076 608
- JP-A- S57 106 842
- US-A1- 2005 098 713
- US-A1- 2010 053 628

## Description

### TECHNICAL FIELD

This invention relates to a plant wavelength sensor device for determining a growth status of a plant or the like.

### BACKGROUND ART

In order to improve the productivity of agricultural products, it is important to accurately determine the growth status of a crop so as to efficiently produce the crop. To this end, there is a known technique that irradiates a crop (e.g., plant (target plant)) with a measurement light, receives the measurement light reflected on the crop, calculates a reflection rate at which the crop reflects the measurement light, and determines, based on the calculated reflection rate, a normalized difference vegetation index (NDVI) that is indicative of the growth status of the crop.

In a conventional technique, a plant wavelength sensor device capable of appropriately receiving the light reflected on the crop to determine the normalized difference vegetation index accurately and to appropriately determine the growth status of the crop has been taught (*see* PTL 1, for example). The conventional plant wavelength sensor device can reduce the influence of a light volume component caused by a disturbance light and appropriately receive the reflected light form the crop. As a result, the conventional plant wavelength sensor device can accurately determine the normalized difference vegetation index to appropriately determine the growth status of the crop. Document US 2010/053628 A1 relates to a plant sensor including a light source section having first and second light emitters configured to irradiate the first and second measuring light toward the object to be measured, respectively, and a light receiver configured to receive reflected light from the object to be measured. This document does not disclose a the light receiving portion including n light receiving members for respectively receiving reflected lights of n wavelength ranges recorded in a control section. Further prior art is described, e.g., in document US 2005/098713 A1.

### CITATION LIST

### Patent Literature

Patent Literature 1: JP2012-247235A

### SUMMARY

### Technical Problem

There is a known technique that determines a content of a specific growth parameter of respective growth parameters such as nutrition and water in a crop (plant (target plant)) based on a reflection rate and a reflected light volume with respect to a light (measurement light) having a specific wavelength range. Accordingly, in a crop (plant (target plant), it is possible to change the wavelength range and the number of wavelength ranges of the measurement light in accordance with a type of a growth parameter of the crop (plant (target plant)).

However, the above plant wavelength sensor device receives the light reflected on the crop (plant (target plant)) with lights having two fixed wavelength ranges set as the measurement lights, and calculates the reflection rate and the reflected light volume at which the crop reflects the measurement lights. For this reason, the above plant wavelength sensor device is inapplicable to various growth parameters of a crop (plant (target plant)), and needs an improvement.

In view of the above problem, an object of the present invention is to provide a plant wavelength sensor device applicable to various growth parameters of a target plant.

### Solution to Problem

A plant wavelength sensor device according to the invention is defined in claim 1.

### Advantageous Effects

The plant wavelength sensor device according to the present invention is applicable to various growth parameters of target plants.

### BRIEF DESCRIPTION OF DRAWINGS

[Fig. 1] Fig. 1 is a perspective view schematically illustrating a plant wavelength sensor device 10 as one example of the plant wavelength sensor device described herein.
[Fig. 2] Fig. 2 is an explanatory view illustrating an arrangement in which two plant wavelength sensor devices 10 are provided to a tractor TR.
[Fig. 3] Fig. 3 is a block diagram schematically showing a functional configuration of the plant wavelength sensor device 10.
[Fig. 4] Fig. 4 is an explanatory view illustrating a configuration of an irradiation optical system 60.
[Fig. 5A] Fig. 5A is an explanatory view illustrating that an irradiation area IA formed by the two plant wavelength sensor devices 10 provided to the tractor TR rotates around an irradiation optical axis L.
[Fig. 5B] Fig. 5B is an explanatory view illustrating that the irradiation area IA formed by the two plant wavelength sensor devices 10 provided to the tractor TR rotates around the irradiation optical axis L.
[Fig. 6] Fig. 6 is a timing chart showing operations (output of various signals and operations along with the output) in the plant wavelength sensor device 10 when estimating the amount of protein (containing water) as an example of a growth parameter Eg.
[Fig. 7A] Fig. 7A is an explanatory view for explaining an example of integration process of light receiving values outputted from a light receiving unit 22 (light receiving member 31).
[Fig. 7B] Fig. 7B is an explanatory view for explaining an example of the integration process of the light receiving values outputted from the light receiving unit 22 (light receiving member 31).
[Fig. 8] Fig. 8 is a flowchart showing a reflection measurement process which is executed by a control section (CPU 23).
[Fig. 9] Fig. 9 is a block diagram schematically illustrating a functional configuration of a plant wavelength sensor device 10A of a Second Embodiment.
[Fig. 10] Fig. 10 is a timing chart showing operations (output of various signals and operations along with the output) in the plant wavelength sensor device 10A when estimating the amount of protein (containing water) as an example of the growth parameter Eg.
[Fig. 11] Fig. 11 is a flowchart showing the reflection measurement process which is executed by the control section (CPU 23) of the plant wavelength sensor device 10A.

### DESCRIPTION OF EMBODIMENT

Hereinafter, embodiments of a plant wavelength sensor device according to the present invention will be described with reference to the drawings.

### First Embodiment

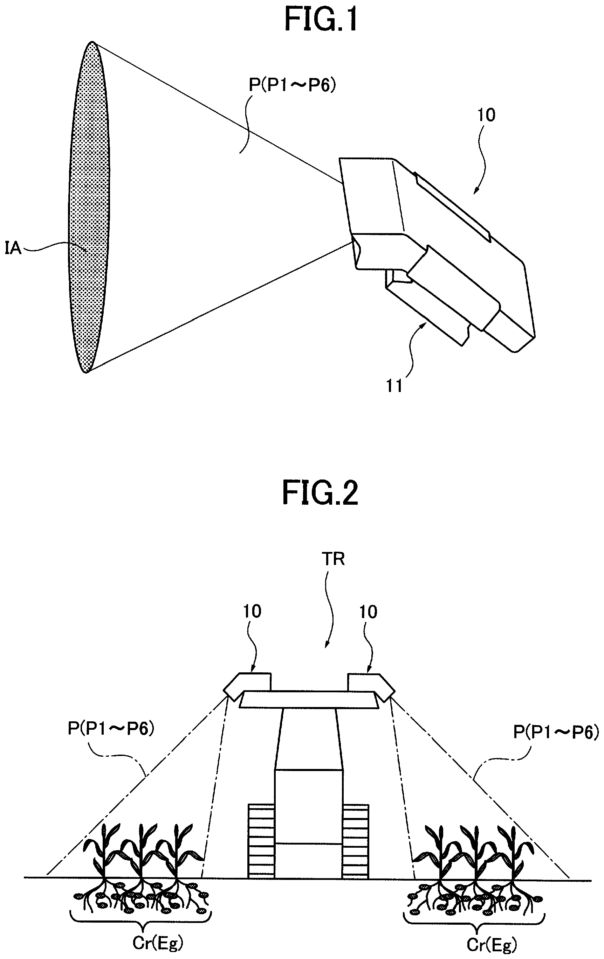

First, a schematic configuration of a plant wavelength sensor device 10 as one embodiment is described with reference to Figs. 1 to 8.

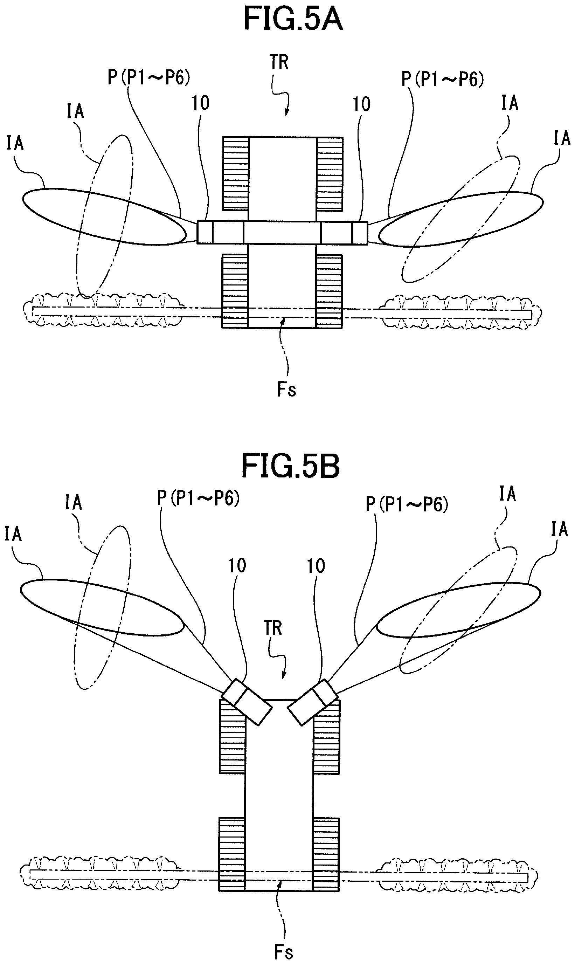

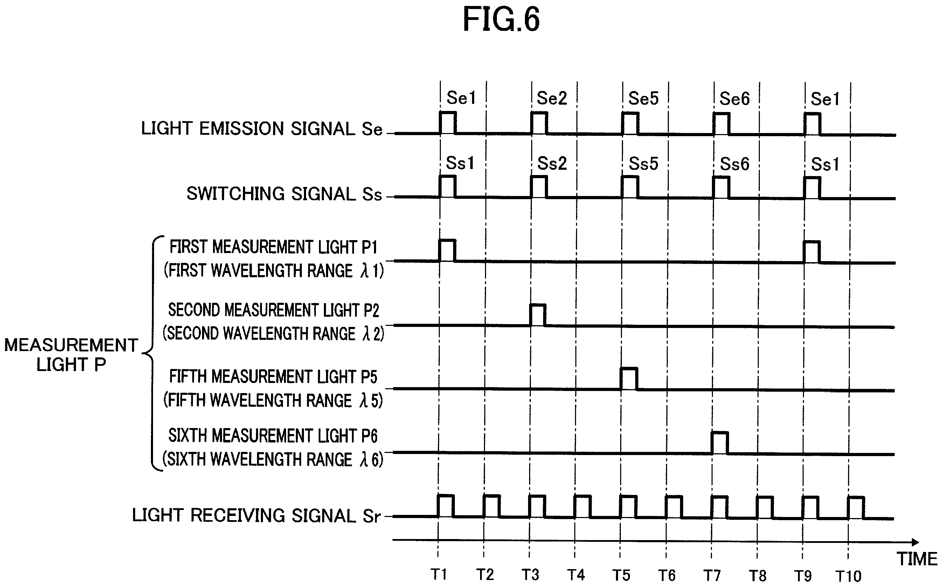

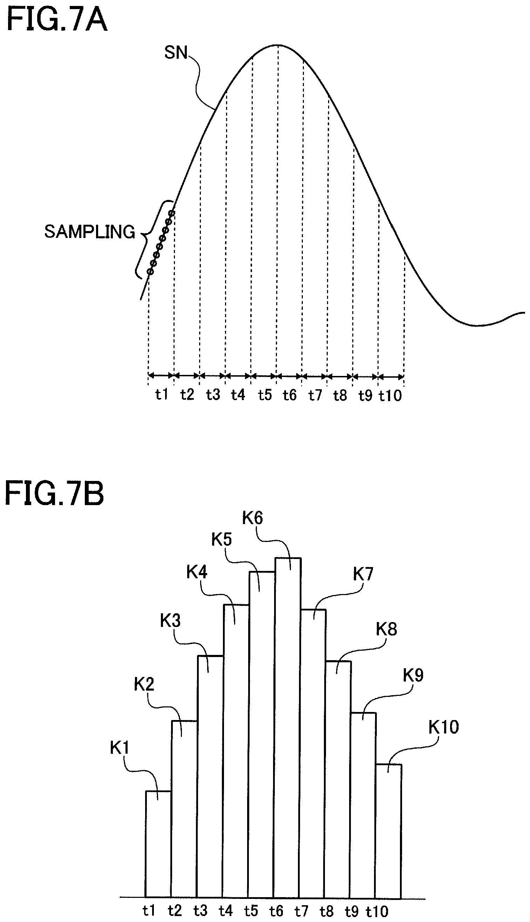

Fig. 5A illustrates two plant wavelength sensor devices 10 arranged on both sides of a tractor TR and Fig. 5B illustrates the two plant wavelength sensor devices 10 diagonally arranged on a front side of the tractor TR. Fig. 7A shows a sampling method for the light receiving output in which the light receiving output is divided into several time-segments and is sampled in each segment, and Fig. 7B shows that sampling values in each time-segment are summed up and the largest value (*i*.*e*., the value K6 in Fig. 7B) among the values of all the segments is determined as a light receiving output value. In addition, although Fig. 6 shows various output signals and measurement lights P as rectangular waves for easily understanding these, the output signals and the measurement lights P do not always coincide with those in the embodiment. Here, each of the measurement lights P is emitted from each light emission member 26 as an operation in response to the output signal.

As illustrated in Fig. 1, the plant wavelength sensor device 10 of the First Embodiment irradiates an irradiation area IA with a plurality of measurement lights P having freely-selected wavelength ranges. The wavelength of the measurement light P means the wavelength at which the spectral intensity of the corresponding measurement light shows its peak value. The plant wavelength sensor device 10 irradiates target plants Cr (*i.e*., plants the growth status of which is determined) with the measurement light P having a specific wavelength range and obtains a reflected light Pr of the measurement light P from the target plant. The plant wavelength sensor device 10 then calculates, as a reflected light volume, the volume of the reflected light Pr of the target plant and the reflection rate of the corresponding target plant with respect to a specific wavelength range to determine (estimate) the content of a growth parameter Eg (*see* Fig 2) corresponding to the specific wavelength range.

The wavelength range and the number of wavelength ranges to be used as the measurement light are changed in accordance with the type of the growth parameter Eg of the target plant. The plant wavelength sensor device 10 is configured to preset a plurality of growth parameters Eg which can be used as measurement targets, and to preset a plurality of wavelength ranges required for estimating each of the set growth parameters Eg. As an example, the plant wavelength sensor device 10 is configured to set nitrogen, protein, and water associated with protein as the growth parameters Eg. The plant wavelength sensor device 10 is configured to set two wavelength ranges of 735 nm and 808 nm for estimating nitrogen, three wavelength ranges of 480 nm, 700 nm, and 1050 nm for estimating protein, and one wavelength range of 950 nm for estimating water associated with the estimation of the protein. Hereinafter, the set wavelength ranges, 480nm, 700nm, 735nm, 808nm, 950nm, and 1050nm are referred to as a first wavelength range λ1, a second wavelength range λ2, a third wavelength range λ3, a fourth wavelength range λ4, a fifth wavelength range λ5, and a sixth wavelength range λ6, respectively, in increasing order. The information is appropriately readably stored in a memory (not shown) to be registered in a CPU 23 (control section).

The plant wavelength sensor device 10 (control section) quantitatively analyzes the growth parameter Eg as follows. In the First Embodiment, the target plant is wheat, and the plant wavelength sensor device 10 quantitatively analyzes protein as a component contained in the wheat. The plant wavelength sensor device 10 emits the measurement lights having the first wavelength range λ1, the second wavelength range λ2, and the sixth wavelength range λ6, which are used for estimating the amount of protein, and emits the measurement light P having the fifth wavelength range λ5 as an absorption wavelength range of water which is used for estimating the amount of protein.

In the First Embodiment, the first wavelength range λ1, the second wavelength range λ2, the fifth wavelength range λ5, and the sixth wavelength range λ6 obtain many spectral distribution curves (optical spectrum (data)) of the reflected lights from the wheat. The first wavelength range λ1, the second wavelength range λ2, the fifth wavelength range λ5, and the sixth wavelength range λ6 are determined by adopting a Partial Least Squares (PLS) regression analysis method to the data. A calibration curve is drawn based on multiple regression analysis with a true value of the amount of protein (a value of the amount of protein obtained with the analysis method for use in the known quantitative analysis of the protein) and the determined first wavelength range λ1, second wavelength range λ2, fifth wavelength range λ5, and sixth wavelength range λ6, so as to determine estimation coefficients regarding the protein. A calibration curve is also drawn based on multiple regression analysis with a true value of the amount of water (a value of the amount of water obtained with the analysis method for use in the known quantitative analysis of water) and the determined first wavelength range λ1, second wavelength range λ2, fifth wavelength range λ5, and sixth wavelength range λ6, so as to determine the estimation coefficients regarding the amount of water. In the First Embodiment, as a wavelength range near the fifth wavelength range λ5 of 950 nm is mainly affected by the amount of water, the fifth wavelength range λ5 is weighted, and the estimation coefficients regarding the amount of water are determined with all of the first wavelength range λ1, the second wavelength range λ2, the fifth wavelength range λ5, and the sixth wavelength range λ6.

The plant wavelength sensor device 10 (control section) estimates the amount of protein as the growth parameter Eg with the volume of reflected light from the wheat (target plant) with respect to the first wavelength range λ1, the second wavelength range λ2, the fifth wavelength range λ5, and the sixth wavelength range λ6 and the estimation coefficients calculated by the multiple regression analysis. This process is similarly used when the nitrogen as the component contained in the target plant is quantitatively analyzed. Accordingly, the plant wavelength sensor device 10 (control section) quantitatively analyzes the growth parameter Eg contained in the target plant.

Additionally, the plant wavelength sensor device 10 (control section) calculates the reflection rate of the target plant with respect to each of the measurement light P having the third wavelength range λ3 and the measurement light P having the fourth wavelength range λ4 to determine a spectroscopy vegetation index of the target plant. The calculation of the reflection rates of the reflected lights Pr will be described in details later. The plant wavelength sensor device 10 (control section) determines, as an example of the spectroscopy vegetation index, a normalized difference vegetation index (NDVI) that is indicative of the growth status of the target plant (the quantity of nutrients in the target plant). To this end, the plant wavelength sensor device 10 (control section) uses a light of a red light wavelength range as the measurement light P having the third wavelength range λ3 and a light of an infrared wavelength range as the measurement light P having the fourth wavelength range λ4. Then, the plant wavelength sensor device 10 (control section) determines the normalized difference vegetation index (NDVI) based on an equation of "NDVI = (IR-R)/(IR+R)", where R represents the reflection rate of the measurement light P of the red light wavelength range (third wavelength range λ3) and IR represents the reflection rate of the measurement light P of the infrared wavelength range (fourth wavelength range λ4).

The plant wavelength sensor device 10 is equipped with an operation section (not illustrated) to perform and realize each function of the device. For example, the functions of the plant wavelength sensor device include the irradiation of each measurement light P, the adjustment of the rotating postures of the irradiation area IA, the calculation (estimation) of the content of the selected growth parameter Eg, and the calculation of the normalized difference vegetation index (NDVI). The plant wavelength sensor device 10 is provided with an attachment part 11. The attachment part 11 allows the plant wavelength sensor device 10 to be attached to any place.

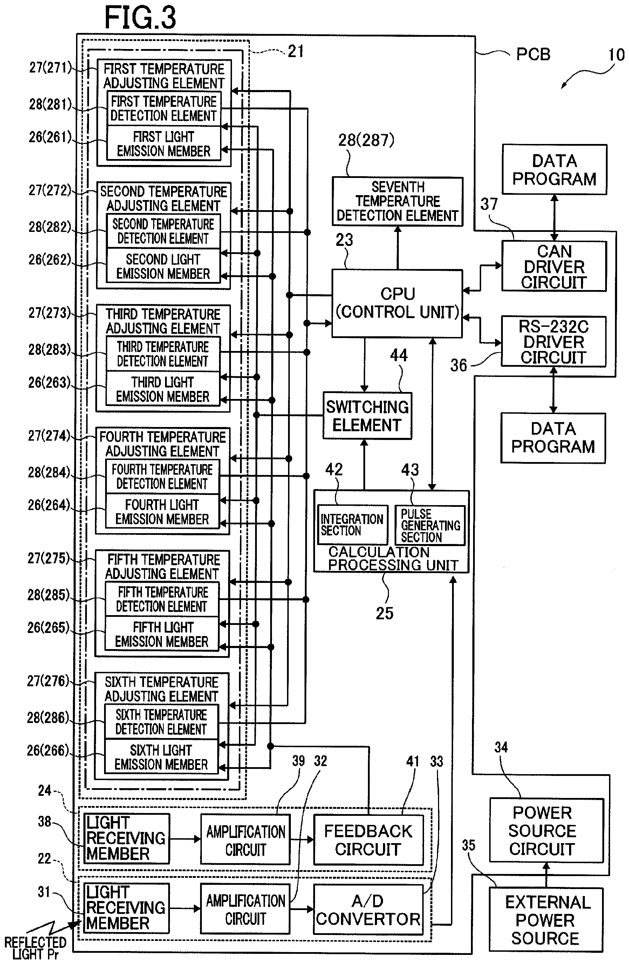

As illustrated in Fig. 2, the plant wavelength sensor device 10 may be provided to an agricultural machine such as a tractor TR via the attachment part 11, for example. In this example of Fig. 2, two plant wavelength sensor devices 10 are respectively provided to both sides of the tractor TR and respectively form the irradiation areas IA on the sides of the tractor TR (*see* Figs. 1, 5A and 5B). When the tractor TR travels, the plant wavelength sensor devices 10 form the irradiation areas IA on the both sides of the tractor TR. That is, by driving the tractor TR along a side of the target plant such as crops Cr, the plant wavelength sensor device 10 can obtain the normalized difference vegetation index (spectroscopy vegetation index) and the content of each growth parameter Eg of the crop Cr. The illustrated tractor TR is further equipped with a fertilizer spreader Fs (illustrated in Figs. 5A, 5B). The fertilizer spreader Fs adjusts the amount of a fertilizer and spreads the fertilizer under the control of a control section (not illustrated). The fertilizer spreader Fs is configured such that the control section thereof communicates and exchanges data with each of the plant wavelength sensor devices 10 (specifically, with its CPU 23) through a driver circuit 36 and a driver circuit 37 *(see* Fig. 3), and spreads the fertilizer in accordance with the normalized difference vegetation index (spectroscopy vegetation index) and the content of each growth parameter Eg obtained by each of the plant wavelength sensor devices 10. Accordingly, the fertilizer spreader Fs can spread the appropriate amount of the fertilizer in accordance with the growth status of the crop Cr while driving the tractor TR along an agricultural land (filed). With this, it becomes possible to cultivate the crop efficiently.

As shown in Fig. 3, the plant wavelength sensor device 10 includes a light emitting unit 21 as an example of a light emitting portion, a light receiving unit 22 as an example of a light receiving portion, the CPU (control unit) 23, an APC unit 24, and the arithmetic processing unit 25. The light emitting unit 21 is a light emitting portion that emits each measurement light P, and includes a plurality of light emission members 26 corresponding to a plurality of wavelength ranges required for estimating the set growth parameters Eg. Namely, the light emitting unit 21 includes n-light emission members 26 corresponding to the registered (set) n-wavelength ranges where n represents a natural number greater than or equal to three (3).

In the First Embodiment, as the six wavelength ranges are set, the light emitting unit 21 includes, as an example of the light emission member, a first light emission member 261, a second light emission member 262, a third light emission member 263, a fourth light emission member 264, a fifth light emission member 265, and a sixth light emission member 266. The light emitting unit 21 is provided with a temperature adjusting element 27 (a first temperature adjusting element 271, a second temperature adjusting element 272, a third temperature adjusting element 273, a fourth temperature adjusting element 274, a fifth temperature adjusting element 275, and a sixth temperature adjusting element 276) and a temperature detection element 28 (a first temperature detection element 281, a second temperature detection element 282, a third temperature detection element 283, a fourth temperature detection element 284, a fifth temperature detection element 285, and a sixth temperature detection element 286) in accordance with each of the light emission members 26.

The first light emission member 261 emits the measurement light P having the first wavelength range λ1. The first light emission member 261 of the First Embodiment includes a pulse-type laser diode (PLD) and the wavelength at the peak value of the emitted light is set to 480 nm. The second light emission member 262 emits the measurement light P having the second wavelength range λ2. The second light emission member 262 of the First Embodiment includes a pulse-type laser diode (PLD) and the wavelength at the peak value of the emitted light is set to 700 nm. The third light emission member 263 emits the measurement light P having the third wavelength range λ3. The third light emission member 263 of the First Embodiment includes a pulse-type laser diode (PLD) and the wavelength at the peak value of the emitted light is set to 735 nm. The fourth light emission member 264 emits the measurement light P having the fourth wavelength range λ4. The fourth light emission member 264 of the First Embodiment includes a pulse-type laser diode (PLD) and the wavelength at the peak value of the emitted light is set to 808 nm. The fifth light emission member 265 emits the measurement light P having the fifth wavelength range λ5. The fifth light emission member 265 of the First Embodiment includes a pulse-type laser diode (PLD) and the wavelength at the peak value of the emitted light is set to 950 nm. The sixth light emission member 266 emits the measurement light P having the sixth wavelength range λ6. The sixth light emission member 266 of the First Embodiment includes a pulse-type laser diode (PLD) and the wavelength at the peak value of the emitted light is set to 1050 nm.

As described later, each of the light emission members 26 (261 to 266) is controlled (to adjust the output and to turn on/off the light) by the output power controller *(i.e.,* APC unit 24 and arithmetic processing unit 25). As described above, each of the temperature adjusting elements 27 (271 to 276) and each of the temperature detection elements 28 (281 to 286) are provided to separately adjust the temperature of each of the light emission members 26.

Each of the temperature adjusting elements 27 heats or cools the corresponding each of the light emission members 26. Each of the temperature adjusting elements 27 is made of a Peltier element. Each of the temperature adjusting elements 27 has a size and a shape that allow each of the light emission members 26 to be installed thereon. Each of the temperature detection elements 28 detects the temperature of the corresponding each of the light emission members 26. In the First Embodiment, each of the temperature detection elements 28 is made of a thermistor.

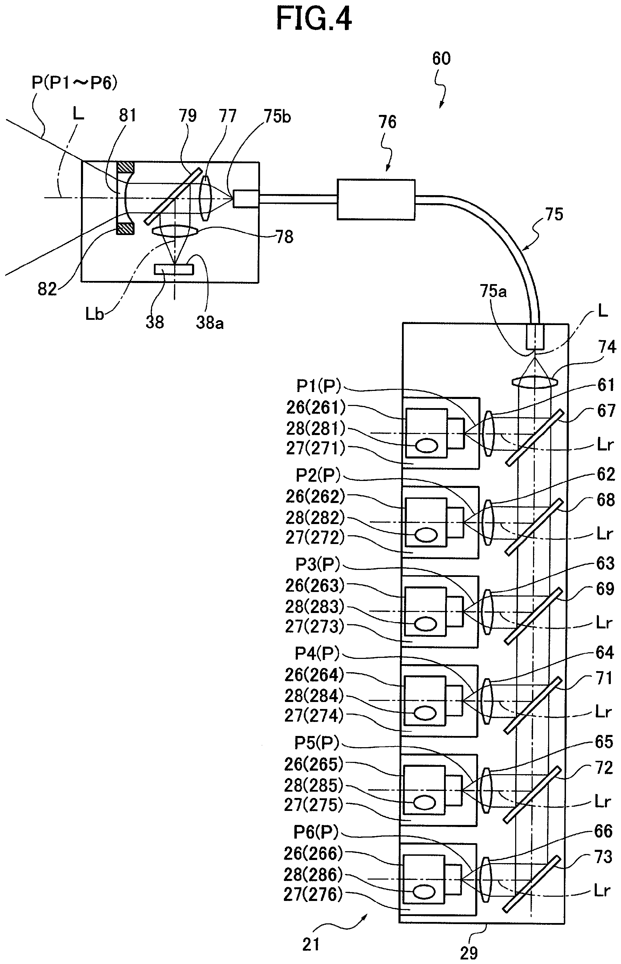

As illustrated in Fig. 4, in the light emitting unit 21 of the First Embodiment, each of the temperature adjusting elements 27 is attached to a base substrate 29 and each of the light emission members 26 is provided to each of the temperature adjusting elements 27 through a metal plate. Each of the temperature detection elements 28 is attached to each of the corresponding light emission members 26. Here, the light emitting unit 21 of the First Embodiment includes a thermistor to detect a temperature of a printed circuit board PCB having various circuits as a seventh temperature detection element 287, as illustrated in Fig. 3. The seventh temperature detection element 287 may be provided at a position close to the light receiving portion (*i.e*., light receiving unit 22 (light receiving member 31) or APC unit 24 (light receiving member 38)) to detect the temperature of the printed circuit board PCB having the driving circuit of the light receiving portion, and the detected temperature is used to correct a light receiving value calculated by the light receiving portion.

In the light emitting unit 21, the detection results of each temperature detection element 28 are inputted to the CPU 23. The CPU 23 then controls the temperature adjusting element 27 to keep the temperature of each of the light emission members 26 constant based on the detection results of each of the temperature detection elements 28. Thus, the CPU 23 functions as the temperature control circuit for controlling the temperature of each of the light emission members 26. Each of the temperature adjusting elements 27 of the First Embodiment is made of the Peltier element. Therefore, by controlling the direction of the current flowing in the Peltier element by the CPU 23, each of the temperature adjusting elements 27 can adjust the temperature.

The light receiving unit 22 includes the light receiving member 31 as an example of a light receiving member, an amplification circuit 32, and an analogue and digital (A/D) convertor 33. The light receiving member 31 receives the reflected light Pr from the target plant (crop Cr) to which each measurement light P is irradiated, and outputs an electrical signal in accordance with the light volume of the received light. The light receiving member 31 of the First Embodiment is formed of six photodiodes PD (not illustrated) and outputs the electrical signals (*i*.*e*., detection result (light receiving value)) to the amplification circuit 32. Note the electrical signals outputted from the light receiving member 31 contain not only the signals generated in accordance with the light volume of the reflected light Pr from the target plant (crop Cr) but also signals generated in accordance with a light volume of a disturbance light. The amplification circuit 32 amplifies the inputted electrical signals appropriately and then outputs the amplified signals to the A/D converter 33. The A/D converter 33 converts the inputted electrical signals (light receiving value) to digital signals and outputs the digital signals to the arithmetic processing unit 25. The light receiving unit 22 receives the reflected light Pr in accordance with the light receiving signal Sr (*see* Fig. 6). The light receiving signals Sr are outputted to the light receiving unit 22 in accordance with a light emission signal Se (*see* Fig. 6). That is, the light receiving signals Sr are outputted to the light receiving unit 22 in accordance with the light emitting signals Se (*see* Fig. 6) under the control of the CPU 23. The light receiving signal Sr is outputted when the light emission signal Se is outputted, and the light receiving signal Sr is also outputted between the light emission signal Se and the next light emission signal Se as the light receiving unit 22 receives the light receiving value when each of the light emission members 26 turns on and when the light emission member 26 turns off.

The Central Processing Unit (CPU) 23 of the plant wavelength sensor device 10 functions as a control unit. That is, the CPU 23 integrally controls each section, to which the electrical power is supplied from an external power source 35 through a power circuit 34, based on a program stored in a memory (not illustrated). The CPU 23 may communicate and exchange data with the outside through the driver circuit 36 and/or the driver circuit 37 to obtain data and/or program necessary to drive the plant wavelength sensor device 10. Here, the driver circuit 36 complies with the RS-232C standard, and the driver circuit 37 is adopted to realize CAN communication. As described above, the CPU 23 can also adjust the temperatures of each of the light emission members 26 (in other words, the CPU can control each of the temperature adjusting elements 27). Further, the CPU 23 switches an electrical circuit (connection destination) of a switching element 44 by outputting a switching signal Ss (*see* Fig. 6) to the switching element 44.

The CPU 23 functions as an arithmetic section for calculating reflection rates and the reflected light volume of the target plant (crop Cr) with respect to the emitted each measurement light P based on the integrated signals outputted from the arithmetic processing unit (integration section 42). The CPU 23 also functions as an arithmetic section for calculating the normalized difference vegetation index (NDVI) of the target plant (crop Cr) based on the calculated results (*i.e*., calculated reflection rates).

The APC unit 24 stabilizes the output power of each light emission member 26. Specifically, the APC unit 24 stabilizes and maintains the intensities (light volumes) of each measurement light P to be outputted at predetermined intensities (feedback control). The APC unit 24 includes the light receiving member 38, the amplification circuit 39, and the feedback circuit 41. The light receiving member 38 is provided to an irradiation optical system 60 (*see* Fig. 4) to receive a part of each measurement light P prior to the emission of the plant wavelength sensor device 10 (specifically, cylindrical lens 81 as emission section). The light receiving member 38 outputs electrical signals in accordance with the light volume of the received light. In the First Embodiment, the light receiving member 38 is formed of a photodiode PD. The light receiving member 38 outputs the electrical signals (detection results (light receiving value)) to the amplification circuit 39. The amplification circuit 39 amplifies and outputs the inputted electrical signals to the feedback circuit 41. Based on the inputted electrical signals, the feedback circuit 41 controls the driving current of each light emission member 26 to keep the light receiving values constant. With this, each light emission member 26 is automatically controlled such that the intensities of each measurement light P emitted from the light emission member 26 is kept constant.

In the APC unit 24, the light receiving member 38 functions as an auxiliary light receiving element that receives a part of the measurement light P irradiated from each of the emission members 26 to the target plant (crop Cr). Further, the amplification circuit 39 and the feedback circuit 41 of the APC unit 24 function as a light volume controller that controls the output powers of each of the light emission members 26 based on the light receiving value outputted from the auxiliary light receiving element such that the intensity of each measurement light P is kept constant.

The arithmetic processing unit 25 is formed of a Field Programmable Gate Array (FPGA), and has an integrating function (integration section 42) and an emission light control function (pulse generating section (emission controller) 43). The integration section 42 integrates the light receiving values of the light receiving member 31 for a predetermined time to generate and output an integrated signal. The pulse generating section 43 generates pulse signals and outputs the generated pulse signals to control each light emission member 26. The operation of the integration section 42 will be described later. The pulse generating section 43 generates pulse signals (light emission signals Se) and outputs the generated pulse signals to each light emission member 26 to control each light emission member 26 under the control of the CPU 23 such that each light emission member 26 emits the light at different timings. In the First Embodiment, the switching element 44 is provided to output the pulse signals generated by the pulse generating section 43 (arithmetic processing unit 25) to the respective light emission members 26.

The switching element 44 selects one of the light emission members 26, and connects the selected light emission member 26 to the pulse generating section 43 (arithmetic processing unit 25). The switching element 44 connects the light emission member 26 corresponding to the switching signal Ss inputted from the CPU 23 to the pulse generating section 43 (arithmetic processing unit 25). The switching signal Ss corresponding to the first light emission member 261 is set as a first switching signal Ss1, the switching signal Ss corresponding to the second light emission member 262 is set as a second switching signal Ss2, the switching signal Ss corresponding to the third light emission member 263 is set as a third switching signal Ss3, the switching signal Ss corresponding to the fourth light emission member 264 is set as a fourth switching signal Ss4, the switching signal Ss corresponding to the fifth light emission member 265 is set as a fifth switching signal Ss5, and the switching signal Ss corresponding to the sixth light emission member 266 is set as a sixth switching signal Ss6. When the switching element 44 receives the n-th switching signal Ssn, the switching element 44 selects an electrical circuit which connects the pulse generating section 43 (arithmetic processing unit 25) and the n-th light emission member 26n to flow current in the selected electrical circuit and not to flow current in the other electrical circuits.

Therefore, the arithmetic processing unit 25 (pulse generating section 43) functions as the output power controller that controls driving (adjusting the output and turning on/off the light) of each light emission member 26 in corporation with the switching element 44, the CPU 23, and the APC unit 24. The output power controller (arithmetic processing unit 25) of the First Embodiment controls each of the light emission members 26 corresponding to the wavelength range set for the selected growth parameter Eg to emit in order at the same time width, and sets equal driving stop (turn off) times after each emission.

Accordingly, in the plant wavelength sensor device 10 of the First Embodiment, the CPU 23, the APC unit 24, and the arithmetic processing unit 25 drive and control each light emission member 26 and the light receiving portion (light receiving member 31). The CPU 23, the APC unit 24, and the arithmetic processing unit 25 function as a control section to determine the reflection rate, the reflected light volume, and the spectroscopy vegetation index (normalized difference vegetation index) of the target plant (crop Cr) from each measurement light P and reflected light Pr.

As illustrated in Fig. 4, the plant wavelength sensor device 10 includes the irradiation optical system 60 to form the predetermined irradiation area IA (*see* Figs. 5A, 5B) by each of the light emission members 26. In addition to each of the light emission members 26 of the light emitting unit 21, the irradiation optical system 60 includes a first lens 61 corresponding to the first light emission member 261, a second lens 62 corresponding to the second light emission member 262, a third lens 63 corresponding to the third light emission member 263, a fourth lens 64 corresponding to the fourth light emission member 264, a fifth lens 65 corresponding to the fifth light emission member 265, and a sixth lens 66 corresponding to the sixth light emission member 266. Each of the first to sixth lenses 61 to 66 collimates the measurement light P emitted from the corresponding light emission member 26 (261 to 266) into a luminous flux parallel to the emission optical axis Lr of the light emission member 26. Each of the emission optical axes Lr of the First Embodiment is parallel to each other, and is arranged to orthogonal to each other to set an irradiation optical axis L of the irradiation optical system 60.

The irradiation optical system 60 also includes, on the irradiation optical axis L, a first dichroic mirror 67, a second dichroic mirror 68, a third dichroic mirror 69, a fourth dichroic mirror 71, a fifth dichroic mirror 72, a mirror 73, and a seventh lens 74. The irradiation optical system 60 also includes an optical fiber 75, a mixing section 76, an eighth lens 77, a ninth lens 78, a half mirror 79, a cylindrical lens 81, and the light receiving member 38.

The first dichroic mirror 67 on the irradiation optical axis L is disposed at the intersection of the irradiation optical axis L and the emission optical axis Lr of the first light emission member 261. The first dichroic mirror 67 allows the measurement light P having the wavelength range except the first wavelength range λ1 of the set wavelength ranges to transmit through the first dichroic mirror 67, and reflects the measurement light P having the first wavelength range λ1 from the first light emission member 261 to travel the measurement light P along the irradiation optical axis L. The first dichroic mirror 67 of the First Embodiment includes an optical characteristic that reflects the light having a wavelength rage about 480 nm as the first wavelength range λ1 and allows the light having a wavelength range larger than a wavelength range about 700 nm as the second wavelength range λ2 to transmit through the first dichroic mirror 67. The second dichroic mirror 68 is disposed at the intersection of the irradiation optical axis L and the emission optical axis Lr of the second light emission member 262. The second dichroic mirror 68 allows the measurement light P having a wavelength range except the first wavelength range λ1 and the second wavelength range λ2 of the set wavelength ranges to transmit the second dichroic mirror 68, and reflects the measurement light P having the second wavelength range λ2 from the second light emission member 262 to travel along the irradiation optical axis L. The second dichroic mirror 68 of the First Embodiment includes an optical characteristic that reflects the light having a wavelength range around 700 nm as the second wavelength range λ2 and allows the light having a wavelength range larger than a wavelength range around 735 nm as the third wavelength range to transmit through the second dichroic mirror 68.

The third dichroic mirror 69 is disposed at the intersection of the irradiation optical axis L and the emission optical axis Lr of the third light emission member 263. The third dichroic mirror 69 allows the measurement light P having a wavelength range except the first wavelength range λ1, the second wavelength range λ2, and the third wavelength range λ3 of the set wavelength ranges to transmit through the third dichroic mirror 69, and reflects the measurement light P having the third wavelength range λ3 from the third light emission member 263 to travel along the irradiation optical axis L. The third dichroic mirror 69 of the First Embodiment includes an optical characteristic that reflects the light having a wavelength range around 735 nm as the third wavelength range λ3, and allows the light having a wavelength range larger than a wavelength range around 808 nm as the fourth wavelength range λ4 to transmit through the third dichroic mirror 69. The fourth dichroic mirror 71 is disposed at the intersection of the emission optical axis Lr and the irradiation optical axis L of the fourth light emission member 264. The fourth dichroic mirror 71 allows the measurement light P having the fifth wavelength range λ5 and the sixth wavelength range λ6 of the set wavelength ranges to transmit through the fourth dichroic mirror 71, and reflects the measurement light P having the fourth wavelength range λ4 from the fourth light emission member 264 to travel along the irradiation optical axis L. The fourth dichroic mirror 71 includes an optical characteristic that reflects the light having a wavelength range around 808 nm as the fourth wavelength range λ4 and allows the light having a wavelength range larger than a wavelength range about 950 nm as the fifth wavelength range λ5 to transmit through the fourth dichroic mirror 71.

The fifth dichroic mirror 72 is disposed at the intersection of the emission optical axis Lr and the irradiation optical axis L of the fifth light emission member 265. The fifth dichroic mirror 72 allows the measurement light P having the sixth wavelength range λ6 of the set wavelength ranges, and reflects the measurement light P having the fifth wavelength range λ5 from the fifth light emission member 265 to travel along the irradiation optical axis L. The fifth dichroic mirror 72 of the First Embodiment includes an optical characteristic that reflects the light having a wavelength range around 950 nm as the fifth wavelength range λ5 and allows the light having a wavelength range larger than a wavelength range around 1050 nm as the sixth wavelength range λ6 to transmit through the fifth dichroic mirror 72. The mirror 73 is disposed at the intersection of the emission optical axis Lr and the irradiation optical axis L of the sixth light emission member 266. The mirror 73 reflects the measurement light P having the sixth wavelength range λ6 from the sixth light emission member 266 to travel the measurement light P along the irradiation optical axis L. The mirror 73 of the First Embodiment includes an optical characteristic that reflects a light having any wavelength range.

The first and fifth dichroic mirrors (67 to 72) and the mirror 73 function as an optical path merger that merges the emission optical paths of the respective light emission members 26, and guides the merged measurement lights to the seventh lens 74 along the same irradiation optical axis L. The seventh lens 74 condenses each measurement light P, which has been emitted from each light emission member 26 to travel along the irradiation optical axis L, onto an incidence surface 75a provided in one end of the optical fiber 75. Each of the emission members 26 is appropriately positioned, and is not limited to the position of the First Embodiment. In this case, each optical characteristic (wavelength range for transmission and reflection) of the first to fifth dichroic mirrors (67 to 72) and the mirror 73 are appropriately set in accordance with the position of each light emission member 26.

The optical fiber 75 emits each measurement light P, which has been entered through the incidence surface 75a, from an emission surface 75b provided on the other end of the optical fiber 75. The optical fiber 75 has a function to transmit each measurement light P thereinside while mixing each measurement light P. The optical fiber 75 of the First Embodiment is provided with a mixing section 76 in order to advance the mixing effect. The mixing section 76 executes a mode scrambling processing to induce mutual exchange of optical power among modes in the light guide path of the optical fiber 75. In the First Embodiment, the optical fiber 75 is wounded around a wound member for the mode scrambling processing. The wound member applies the mode scrambling processing by winding the optical fiber 75 within a range of allowable bending radius of the optical fiber 75. As a result, each measurement light P outputted from the optical fiber 75 (*i.e*., emission surface 75b) has the intensity equal to each other when viewed in a plane orthogonal to the light traveling direction, and is made as an unpolarized light (randomly-polarized light). Here, each measurement light P emitted from the corresponding light emission member 26 has an oval shape when viewed in the plane orthogonal to the light traveling direction. However, each measurement light P outputted from the emission surface 75b has a circular shape in accordance with the shape of the emission surface 75b. The optical fiber 75 emits each measurement light P, which has entered through the incidence surface 75a, from the emission surface 75b to the eighth lens 77 along the irradiation optical axis L.

The eighth lens 77 collimates each measurement light P emitted from the emission surface 75b into a luminous flux parallel to the irradiation optical axis L. The half mirror 79 is provided behind the eighth lens 77 on the irradiation optical axis L. The half mirror 79 transmits a part of the inputted collimated luminous flux (*i.e.,* each measurement light P), and reflects the other part of the inputted collimated luminous flux on a branched emission optical axis Lb on which the ninth lens 78 is disposed. The ninth lens 78 condenses the collimated luminous flux (*i.e*., each measurement light P), which has been reflected by the half mirror 79, onto an incidence surface 38a of the light receiving member 38 of the APC unit 24 on the branched emission optical axis Lb. That is, the half mirror 79 functions as a luminous flux splitter that splits a part of each measurement light P toward the light receiving member 38 that functions as the output power controller. With this, the APC unit 24 is capable of adjusting the output power of the corresponding each light emission member 26 by using each measurement light P that is an unpolarized light having a uniform intensity distribution after passing through the optical fiber 75 (the common outgoing light path).

The cylindrical lens 81 is provided behind the half mirror 79 on the irradiation optical path L. The cylindrical lens 81 is an optical member having a relatively strong refractive power only in one direction when viewed in a plane orthogonal to the irradiation optical axis L, and expands each measurement light P through the half mirror 79 in the one direction when viewed in the plane orthogonal to the irradiation optical axis L. As described above, each measurement light P emitted from the emission surface 75b of the optical fiber 75 has a circular shape when viewed in the plane orthogonal to the irradiation optical axis L. Each measurement light P having the circular cross section shape through the half mirror 79 is expanded by the cylindrical lens 81 to a predetermined size only in the one direction, resulting in having an oval cross section shape.

The cylindrical lens 81 is supported to be rotatable about the irradiation optical axis L by a rotation driver 82 (*see* Fig. 4). The rotation driver 82 is fixed to a casing (not illustrated) in which the irradiation optical system 60 of the plant wavelength sensor device 10 is housed. The cylindrical lens 81 forms an emission surface from which each measurement light P is emitted. By rotating the cylindrical lens 81 about the irradiation optical axis L by the rotation driver 82 (*see* Fig. 4), the irradiation optical system 60 can change the direction to expand each measurement light P when viewed in the plane orthogonal to the irradiation optical axis L. Thus, it is possible to rotate the irradiation area IA formed by each measurement light P about the irradiation optical axis L (rotated on its own axis).

In the irradiation optical system 60, each light emission member 26 emits the corresponding each measurement light P in response to light-on control executed by the pulse generating section 43 of the arithmetic processing unit 25. Each measurement light P emitted from each light emission member 26 passes through the corresponding lens (61 to 66), and is reflected by the corresponding dichroic mirror (67 to 72) or the mirror 73. Each measurement light P then travels to the seventh lens 74 along the irradiation optical axis L. Accordingly, in the irradiation optical system 60, the emission (irradiation) optical paths of the respective measurement lights P are merged by the dichroic mirror (67 to 72) and the mirror 73, and then extend to the seventh lens 74 along the irradiation optical system 60. The luminous flux (each measurement light P) passed through the seventh lens 74 is inputted to the optical fiber 75 through the incidence surface 75a, and emitted from the emission surface 75b through a light guide path by the optical fiber 75. The luminous flux then travels to the eighth lens 77. The luminous flux (each measurement light P) passed through the eighth lens 77 along the irradiation optical path L is partially reflected by the half mirror 79. The reflected luminous flux is then inputted to the light receiving member 38 through the ninth lens 78 on the branched emission optical axis Lb. On the other hand, the remaining luminous flux is expanded in the one direction by the cylindrical lens 81 to have the oval cross section shape, and emitted from the cylindrical lens 81. In this way, the optical path extending to the cylindrical lens 81 through the seventh lens 74, the optical fiber 75, the eighth lens 77, and the half mirror 79 forms a common emission optical path that connects the dichroic mirror (67 to 72) and the mirror 73 as the optical path merger and the cylindrical lens 81 as the light emitter defining an emission surface.

With the above configuration, the irradiation optical system 60 is capable of emitting each measurement light P from the same cylindrical lens 81 on the same irradiation optical axis L. Further, the irradiation optical system 60 is capable of forming the same oval-shaped irradiation area IA by each measurement light P. In addition, the irradiation optical system 60 is capable of rotating the irradiation area IA of each measurement light P about the irradiation optical axis L (on the own axis) by appropriately rotating the cylindrical lens 81 using the rotation driver 82 (*see* Fig. 4), as illustrated in Figs. 5A, 5B. The plant wavelength sensor device 10 is capable of adjusting the position to form the irradiation area IA around the tractor TR regardless of how to install the plant wavelength sensor device 10 to the tractor TR. As a result, it is possible to increase the freedom of the installation of the plant wavelength sensor device 10 to the tractor TR. Further, the plant wavelength sensor device 10 is capable of adjusting the position to form the irradiation area IA around the tractor TR even after installing the plant wavelength sensor device 10 to the tractor TR.

Each of the light emission signals Se outputted from the pulse generating section 43 *(see* Fig. 3) of the arithmetic processing unit 25 has an equal pulse time width and sequentially generates at an equal interval to have an equal period (*see* Fig. 6), in order to periodically emit light from each light emission member 26. For this reason, each light emission member 26 emits each measurement light P in order at the equal intervals and the equal time width. The light emission signal Se of the example in Fig. 6 showing the operation for estimating the amount of protein (containing water) includes a first light emission signal Se1, a second light emission signal Se2, a fifth light emission signal Se5, and a sixth light emission signal Se6 each having the equal period. The first light emission member 261 periodically emits light by the first light emission signal Se1, the second light emission member 262 periodically emits light by the second light emission signal Se2, the fifth light emission member 265 periodically emits light by the fifth light emission signal Se5, and the sixth light emission member 266 periodically emits light by the sixth light emission signal Se6. By switching the electrical circuit of the switching element 44 with the switching signal Ss from the CPU 23, the light emission signal Se is sent to the corresponding light emission member 26. When the light emission signal Se is outputted (specifically, just before the light emission light signal Se is outputted), the corresponding switching signal Ss is outputted to the switching element 44. In the example of Fig. 6, the first switching signal Ss1 is outputted to the switching element 44, the first light emission signal Se1 is outputted to the first light emission member 261, the second switching signal Ss2 is outputted to the switching element 44, the second light emission signal Se2 is outputted to the second light emission member 262, the fifth switching signal Ss5 is outputted to the switching element 44, the fifth light emission signal Se5 is outputted to the fifth light emission member 265, the sixth switching signal Ss6 is outputted to the switching element 44, and the sixth light emission signal Se6 is outputted to the sixth light emission member 266.

As described above, the integration section 42 of the arithmetic processing unit 25 integrates the light receiving values from the light receiving unit 22 (specifically, the light receiving member 31 thereof (*see* Fig. 3)) for a predetermined time period, and outputs the integrated signals. The light receiving unit 22 (light receiving member 31) receives the light when each light emission member 26 emits a light to acquire or receive a light volume containing the reflected light component of each measurement light P and the disturbance light component generated by the disturbance light. The light receiving unit 22 (light receiving member 31) also receives the light when each light emission member 26 turns off to acquire or receive a light volume containing the disturbance light component excluding the reflected light component of each measurement light P. As a result, the light receiving unit 22 periodically outputs a light receiving value corresponding to the light volume containing the reflected light component of each measurement light M and the disturbance light component and the light receiving value corresponding to the light volume containing only the disturbance light component. When the light receiving unit 22 receives the reflected light value, the integration section 42 executes a first integration step to synchronize with the light-on control of each light emission member 26 and a second integration step to synchronize with the light-off control of each light emission member 26. Here, the light-on control and the light-off control are both executed by the pulse generating section 43. An example of the integration process executed by the integration section 42 (arithmetic processing unit 25) will be described hereinafter.

For example, the integration section 42 divides the pulse width of the light receiving value SN, which is a pulse-type light receiving value containing the reflected light component of each measurement light P and the disturbance light component, into segments t1 to t10, which are equal to each other, as shown in Fig. 7A. The integration section 42 then takes several samples from the light receiving outputs and sums up (or integrates) the sampled light receiving outputs for each segment (t1 to t10), and stores the sum values temporally. For example, the integration section 42 takes eight samples from the light receiving outputs and sums up the sampled eight light receiving outputs in the segment t1 to obtain the sum value K1, as shown in Fig. 7B, and then stores the sum value K1 temporally. Similarly, the integration section 42 acquires sum values K2 to K10 for the other sections t2 to t10. The integration section 42 then selects the largest sum value among the sum values K1 to K10 and determines the selected sum values as the light receiving output value that represents a peak value (*i*.*e*., largest value) of the light receiving value. In the example shown in Fig. 7B, the sum value K6 is determined to be the light receiving output value (*i.e.,* the peak value of the light receiving value SN (*see* Fig. 7A)). The integration section 42 (arithmetic processing unit 25) obtains the light receiving output value (peak value) for each of the several light receiving values obtained within a predetermined time, integrates a predetermined number of the light receiving output values (peak values) of each light receiving value to acquire a first integrated signal. With the first integrated signal, the reflected light component generated by the corresponding measurement light P is emphasized. Here, the process to obtain the first integrated signal represents the first integration step, and the integration section 42 outputs the integration results to the CPU 23.

The integration section 42 (arithmetic processing unit 25) executes a similar calculation for several light receiving values as pulse-type light receiving values, from which the reflected light component of the corresponding measurement light P is excluded. With this, the integration section 42 obtains the light receiving output value (peak value) for each of the several light receiving values, and integrates a predetermined number of light receiving output values (peak value) to acquire a second integrated signal. As the reflected light component of the measurement light P has been excluded, the second integrated signal is the signal representing the disturbance light only. Here, the process to obtain the second integrated signal represents the second integration step, and the integration section 42 outputs the integration results to the CPU 23.

The CPU 23 receives, from the arithmetic processing unit 25 (specifically, from the integration section 42 thereof), the first integrated signal and the second integrated signal. The CPU 23 then subtracts the second integrated signal from the first integrated signal to calculate a reflected light receiving value (reflected light volume) representing the reflected light component of the measurement light P from which the light volume component generated by the disturbance light has been excluded. The CPU 23 also calculates a reflection rate of the target plant (crop Cr), to which the corresponding measurement light P is emitted based on the total light volume of the light emission member 26 which has emitted the corresponding measurement light P and the reflected light receiving value calculated as described above. The total light volume can be determined based on the light emission signal Se set and outputted in each light emission member 26 and the light receiving value by the light receiving member 38. The CPU 23 executes this calculation to each measurement light P to calculate the reflected light volume from the target plant (crop Cr) and the reflection rate of the target plant with respect to each wavelength range. The CPU 23 calculates the normalized difference vegetation index (NDVI) as described above with the obtained reflection rate.

Accordingly, the CPU 23 can obtain the reflected light volume and the reflection rate of each measurement light P which have extremely low light volume component caused by the disturbance light, and can obtain the normalized difference vegetation index (NDVI) of the target plant (crop Cr) based on the reflected light volume and the reflection rate. As a result, it becomes possible to obtain the information regarding the growth status of the target plant (crop Cr). As described above, the information regarding the growth status of the target plant (crop Cr) may be outputted to the outside through the driver circuit 36 and/or the driver circuit 37.

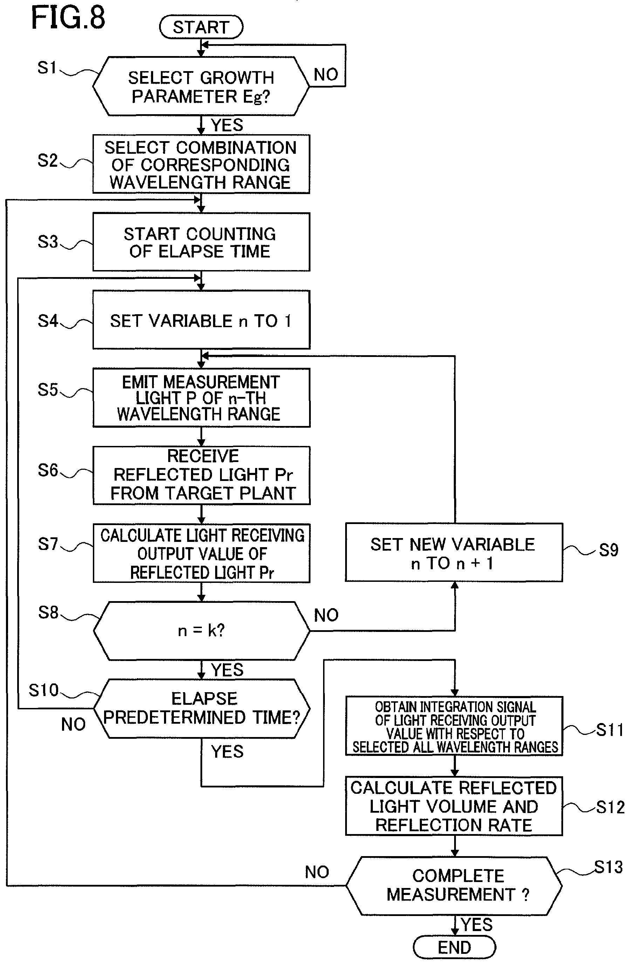

Next, a reflection measurement step of calculating the reflected light volume and the reflection rate of the target plant with respect to the wavelength range for estimating the amount of the growth parameter Eg selected with the plant wavelength sensor device 10 is described with reference to Fig. 8. Fig. 8 is a flowchart showing the reflection measurement process executed by the control section (CPU 23) of the First Embodiment. The control section (CPU 23) executes this reflection measurement process based on the program stored in a memory built in the control section (CPU 23) or provided in the outside of the control section (CPU 23). Hereinafter, each step of the flowchart of Fig. 8 is described. The reflection measurement process in the flowchart of Fig. 8 starts upon the turning on of the plant wavelength sensor device 10.

In step S1, it is determined whether or not the growth parameter Eg is selected. When the determination result is YES, the process proceeds to step S2. When the determination result is NO, step S1 is repeated. In step S1, it is determined whether or not the growth parameter Eg is selected by an operation section (not illustrated). Here, step S1 is repeated until the growth parameter Eg is selected.

In step S2, following the determination that the growth parameter Eg is selected in step S1, a combination of the selected growth parameter Eg and the corresponding wavelength range is selected, and the process proceeds to step S3. In step S2, the combination of the selected growth parameter Eg and the corresponding wavelength range, namely, a plurality of wavelength ranges required for estimating the growth parameter Eg is selected, and the information thereof (the corresponding wavelength ranges and the number thereof) is stored in the memory. In the First Embodiment, as described above, the selectable growth parameters Eg include nitrogen, protein, and water associated with protein. In step S2, when the nitrogen is selected as the growth parameter Eg, the third wavelength range λ3 (735 nm) and the fourth wavelength range λ4 (808 nm) are selected. The third wavelength range λ3 is used as the first wavelength range, the fourth wavelength range λ4 is used as the second wavelength range, and the number of the wavelength ranges k is set to 2 (k = 2). Similarly, in step S2, when the protein (containing water) is selected as the growth parameter Eg, the first wavelength range λ1 (480 nm), the second wavelength range λ2 (700 nm), the fifth wavelength range λ5 (950 nm), and the sixth wavelength range λ6 (1050 nm) are selected. The first wavelength range λ1 is used as the first wavelength range, the second wavelength range λ2 is used as the second wavelength range, the fifth wavelength range λ5 is used as the third wavelength range, the sixth wavelength range λ6 is used as the fourth wavelength range, and the number of wavelength ranges is set to 4 (k = 4). Further, in step S2, when the protein which is not associated with water is selected as the growth parameter Eg, the first wavelength range λ1 (480 nm), the second wavelength range λ2 (700 nm), and the sixth wavelength range λ6 (1050 nm) are selected. The first wavelength range λ1 is used as the first wavelength range, the second wavelength range λ2 is used as the second wavelength range, the sixth wavelength range λ6 is used as the third wavelength range, and the number of wavelength ranges is set to 3 (k = 3).

In step S3, following the selection of the combination of the wavelength ranges in step S2 or the determination that that the measurement has not been completed in step S13, counting of the elapse time for integration of the light receiving values is started. The process then proceeds to step S4. In step S3, the counting of the elapse time of the integration process is started as the integration section 42 (arithmetic processing unit 25) executes the integration process with respect to a plurality of light receiving values received within a predetermined time.

In step S4, following the start of the counting of the elapse time in step S3 or the determination that a predetermined time has not elapsed in step S10, the variable n is set to 1 (n = 1). The process then proceeds to step S5. The variable n is used for counting what number of the wavelength range is subjected for the current emission of the measurement light P and for the current receiving the reflected light Pr. In step S4, the variable n is set to 1.

In step S5, following the setting of the variable n to 1 in step S4 or the new variable n to n + 1 in step S9, the measurement light P having n-th wavelength range is emitted. The process then proceeds to step S6. In step S5, the n-th switching signal Ssn is outputted to the switching element 44 to electrically connect the light emission member 26 corresponding to the n-th wavelength range and the pulse generating section 43 (arithmetic processing unit 25), and to output the n-th light emission signal Sen to the light emission member 26 corresponding to the n-th wavelength range. Accordingly, the measurement light P having the n-th wavelength range is emitted toward the growth target (crop Cr) from the corresponding light emission member 26.

In step S6, following the emission of the measurement light P having the n-th wavelength range in step S5, the reflected light Pr of the measurement light P from the target plant (crop Cr) is received. The process then proceeds to step S7. In step S6, the light receiving unit 22 obtains the light receiving value in accordance with the light receiving signal Sr outputted in response to the n-th light emission signal Sen. In the First Embodiment, when the light emission member 26 corresponding to the n-th wavelength range emits a light and when the light emission member 26 lights off just after the emission, the light receiving signal Sr is outputted. In step S6, the light receiving unit 22 obtains and outputs the light receiving value when the light emission member 26 corresponding to the n-th wavelength range emits a light and the light receiving value when the light emission member 26 lights off just after the emission.

In step S7, following the receiving of the reflected light Pr (its light receiving value) in step S6, the light receiving output value of the received reflected light Pr (light receiving value) is calculated. The process then proceeds to step S8. In step S7, the light receiving output value (first integration step) of the light receiving value when the light emission member 26 corresponding to the n-th wavelength range emits a light and the light receiving output value (second integration step) of the light receiving value when the light emission member 26 lights off just after the emission are calculated.

In step S8, following the calculation of the light receiving output values in step S7, it is determined whether or not the variable n is equal to the number of wavelength ranges k (n = k?). When the determination result is YES, the process proceeds to step S10. When the determination result is NO, the process proceeds to step S9. In step S8, it is determined whether or not the variable n is equal to the set number of wavelength range k. Here, the variable n is used for counting what number of the wavelength range was subjected to the operations from step S5 to step S7. In other word, in step S8, it is determined whether or not the light receiving values with respect to the set all wavelength ranges are obtained.

In step S9, following the determination that the variable n is unequal to the number of wavelength ranges k, the new variable n is set to n + 1. The process then returns to step S5. In step S9, as the light receiving values (light receiving output values) with respect to the set all wavelength ranges are not obtained, the new variable n is set to n + 1 by adding 1 to the variable n (the present value) in order to obtain the light receiving values with respect to the rest of the wavelength ranges.

In step S10, following the determination that the variable n is equal to the number k of wavelength ranges in step S8, it is determined whether or not a predetermine time has elapsed. When the determination result is YES, the process proceeds to step S11. When the determination result is NO, the process returns to step S4. In step S10, it is determined whether or not a predetermined time has elapsed by determining whether or not the predetermined time in step S3 has reached a predetermined time which executes an integration process.

In step S11, following the determination that a predetermined time has elapsed in step S10, the integration signal of the light receiving output values with respect to the selected all wavelength ranges is obtained. The process then proceeds to step S12. In step S11, the integration signals of the light receiving output values with respect to the selected all wavelength ranges, namely, the first integration signal and the second integration signal with respect to the selected all wavelength ranges are obtained. Here, the integration signals are obtained by repeating the operation from step S5 to step S7 for a predetermined time with respect to the selected all wavelength ranges.

In step S12, following the obtaining of the integration signal of the light receiving output values with respect to the selected all wavelength ranges in step S11, the reflected light volume and the reflection rate are calculated. The process then proceeds to step S13. In step S12, the reflected light receiving value (reflected light volume) representing the reflected light component from the target plant (crop Cr) is calculated with respect to the selected all wavelength ranges by deducing the second integration signal from the first integration signal of the light receiving output values obtained in step S11. In step S12, the reflection rate of the target plant (crop Cr) irradiated with the corresponding measurement light P is calculated based on the reflected light receiving value and the total light emission amount of the light emission member 26 emitting the corresponding measurement light P (the value based on the light receiving value in the light receiving member 38, for example). Here, the reflection rate is calculated with respect to the selected all wavelength ranges. The normalized difference vegetation index (NDVI) is appropriately calculated with the calculated reflection rate.

In step S13, following the calculation of the reflected light volume and the reflection rate in step S12, it is determined whether or not the measurement with the plant wavelength sensor device 10 has been completed. When the determination result is YES, the reflection measurement process is completed. When the determination result is NO, the process returns to step S3. In step S13, it is determined whether or not the operation (exit operation) to complete the measurement with the plant wavelength sensor device 10 has been performed. When the operation (exit operation) has not been performed, the above operation to calculate the reflected light volume and the reflection rate is repeated.

Next, the operation of the plant wavelength sensor device 10 for estimating the amount of growth parameter Eg is described with reference to Fig. 6 showing the example in which the protein (containing water) is selected as the growth parameter Eg.

Firstly, when the protein (containing water) is selected as the growth parameter Eg, the process proceeds from S1 to S2 in the flowchart of Fig. 8 to select the first wavelength range λ1 (480 nm), the second wavelength range λ2 (700 nm), the fifth wavelength range λ5 (950 nm), and the sixth wavelength range λ6 (1050 nm). After that, the process proceeds from step S3 to S4 to S5 in the flowchart of Fig. 8 to emit the measurement light P (first measurement light P1) having the first wavelength range λ1 (480 nm) from the first light emission member 261 (*see* time T1 of Fig. 6). The process then proceeds from step S6 to step S7 in the flowchart of Fig. 8 to receive the reflected light Pr from the target plant (crop Cr) with respect to the first wavelength range λ1 (480 nm) and to calculate the light receiving output value (*see* times T1, T2 of Fig. 6). After that, the process proceeds from step S8 to step S9 to step S5 to step S6 to step S7 in the flowchart of Fig. 8 to emit the measurement light P (second measurement light P2) having the second wavelength range λ2 (700 nm) from the second light emission member 262 (*see* T3 of Fig. 6), to emit the measurement light P (third measurement light P3) having the fifth wavelength range λ5 (950 nm) from the fifth light emission member 265 (*see* T5 of Fig. 6), to emit the measurement light P (fourth measurement light P4) having the sixth wavelength range λ6 (1050 nm) from the sixth light emission member 266 (*see* T7 of Fig. 6), to receive the reflected light Pr from the growth target (crop Cr) with respect to each wavelength range (*see* T3 to T8 of Fig. 6), and to calculate the light receiving output value. The process then proceeds from step S8 to step S10 to step S4 in the flowchart of Fig. 8 to repeat the above operation until a predetermined time elapses. When the predetermined time has elapsed, the process proceeds from step S10 to step S11 to step S12 in the flowchart of Fig. 8 to calculate the reflected light receiving value (reflected light volume) representing the reflected light component from the target plant (crop Cr) and the reflection rate of the target plant (crop Cr) with respect to the four wavelength ranges. The amount of the water and the protein contained in the target plant (crop Cr) in the irradiation area IA can be estimated at the predetermined time. After that, until the exit operation, the process proceeds from step S12 to step S13 to step S3 in the flowchart of Fig. 8 to estimate the amount of the water and the protein contained in the target plant (crop Cr) in a next irradiation area IA.

When the nitrogen is selected as the growth parameter Eg, the third wavelength range λ3 (735 nm) and the fourth wavelength range λ4 (808 nm) are selected. The third light emission member 263 emits the measurement light P having the third wavelength range λ3 (735 nm), and the fourth light emission member 264 emits the measurement light P having the fourth wavelength range λ4 (808 nm). The other operation similar to the above is also executed. Accordingly, when the growth parameter Eg is selected, the plant wavelength sensor device 10 can calculate the reflected light volume and the reflection rate of the target plant (crop Cr) with respect to the measurement lights P having a plurality of wavelength ranges required for estimating the selected growth parameter Eg, and can estimate the amount of the growth parameter Eg.

As described above, when the growth parameter Eg is selected, the plant wavelength sensor device 10 of the First Embodiment irradiates the target plant (crop Cr) with the measurement lights P having a plurality of wavelength ranges required for estimating the growth parameter Eg and receives the reflected light Pr of each measurement light P from the target plant (crop Cr). As a result, the plant wavelength sensor device 10 calculates the reflected light volume and the reflection rate of the target plant (crop Cr) having a plurality of wavelength ranges required for estimating the growth parameter Eg. Accordingly, the plant wavelength sensor device 10 can calculate the reflected light volume and the reflection rate of each wavelength range required for estimating the selected growth parameter Eg. Thus, the amount of various growth parameters Eg contained in the target plant (crop Cr) can be estimated.

The plant wavelength sensor device 10 prerecords a plurality of selectable growth parameters Eg and a plurality of wavelength ranges (n) required for estimating the growth parameter Eg, and includes the light emission members 26 corresponding to the number of wavelength ranges registered in the light emitting unit 21 as the light emission portion. Accordingly, the plant wavelength sensor device 10 can easily irradiate the target plant (crop Cr) with the measurement lights P having a plurality of wavelength ranges required for estimating the selected growth parameter Eg.

The plant wavelength sensor device 10 selects a plurality of wavelength ranges required for estimating the selected growth parameters Eg, and emits the measurement light P from the light emission member 26 corresponding to each wavelength range to irradiate the target plant (crop Cr) with the measurement lights P having a plurality of wavelength ranges required for estimating the selected growth parameter Eg. Accordingly, the plant wavelength sensor device 10 can emit the measurement lights P having a plurality of wavelength ranges required for estimating the selected growth parameter Eg by operating the light emission member 26 corresponding to the growth parameter Eg selected in a preset plurality of light emission members 26.

The plant wavelength sensor device 10 sequentially emits a light from a plurality of light emission members 26, namely, emits a light in a chronological order. With this, the plant wavelength sensor device 10 can appropriately and easily receive the reflected light Pr of the measurement light P having each wavelength range from the target plant (crop Cr).

The plant wavelength sensor device 10 prerecords the nitrogen as the selectable growth parameter Eg, and a red light wavelength range (third wavelength range λ3 (735 nm)) and a near-infrared light wavelength range (fourth wavelength range λ4 (808 nm)) as the wavelength ranges required for estimating the nitrogen. Accordingly, the plant wavelength sensor device 10 can appropriately and easily estimate the amount of nitrogen as the growth parameter Eg contained in the target plant (crop Cr).

The plant wavelength sensor device 10 prerecords the protein as the selectable growth parameter Eg, and two different visible wavelength ranges (first wavelength range λ1 (480 nm) and second wavelength range λ2 (700 nm)) and the near-infrared wavelength range (sixth wavelength range λ6 (1050 nm)) as the wavelength ranges required for estimating the protein. Accordingly, the plant wavelength sensor device 10 can easily and appropriately estimate the amount of protein as the growth parameter Eg contained in the target plant (crop Cr).

The plant wavelength sensor device 10 records the protein and the water associated with the estimation of the protein as the selectable growth parameter Eg, and the two different visible wavelength ranges (first wavelength range λ1 (480 nm) and the second wavelength range λ2 (700 nm)) and the near-infrared wavelength range (sixth wavelength range λ6 (1050 nm)) as the wavelength ranges required for estimating the protein. The plant wavelength sensor device 10 records the near-infrared wavelength range (fifth wavelength range λ5 (950 nm)) as the wavelength range required for estimating the water. Accordingly, the plant wavelength sensor device 10 can appropriately and easily estimate the amount of protein and water as the growth parameters Eg contained in the target plant (crop Cr).

The plant wavelength sensor device 10 works as the fertilizer spreader Fs and the fertilization system. The plant wavelength sensor device 10 adjusts the spreading amount of the fertilization of the fertilizer spreader Fs based on the information of the obtained amount of growth parameter Eg to spread the fertilization according to the status of the target plant (crop Cr). With this, the spreading amount of the fertilizer can be adjusted in accordance with the status of the growth parameter Eg in the target growth (crop Cr). Accordingly, the growth of the target plant (crop Cr) can be encouraged while the growth of the target plant (crop Cr) can be limited with the reduced spreading amount of the fertilizer in an area (irradiation area) where the target plant (crop Cr) is well grown. The target plant (crop Cr) can be thereby prevented from falling over by its own weight. The plant wavelength sensor device 10 works as the fertilizer spreader Fs and the fertilization system. Thus, the plant wavelength sensor device 10 can easily and effectively spread the fertilizer to the target plant (crop Cr). With this, when the plant wavelength sensor device 10 is provided to the tractor TR equipped with the fertilizer spreader Fs to work as the fertilization system, the plant wavelength sensor device 10 can spread the appropriate amount of the fertilizer in accordance with the growth states of the target plant to the target plant (crop Cr) by simply traveling the land where the target plants (crop Cr) are grown. Accordingly, the target plant (crop Cr) can be effectively grown.

The plant wavelength sensor device 10 of the First Embodiment is applicable to various growth parameters Eg of the target plant (crop Cr).

In the First Embodiment, the corresponding light emission member 26 and the pulse generating section 43 (arithmetic processing unit 25) are electrically connected by switching the electrical circuit with the switching element 44. However, another configuration may be used as long as it emits the measurement lights having the selected plurality of wavelength ranges (six wavelength ranges in Second Embodiment).

### Second Embodiment

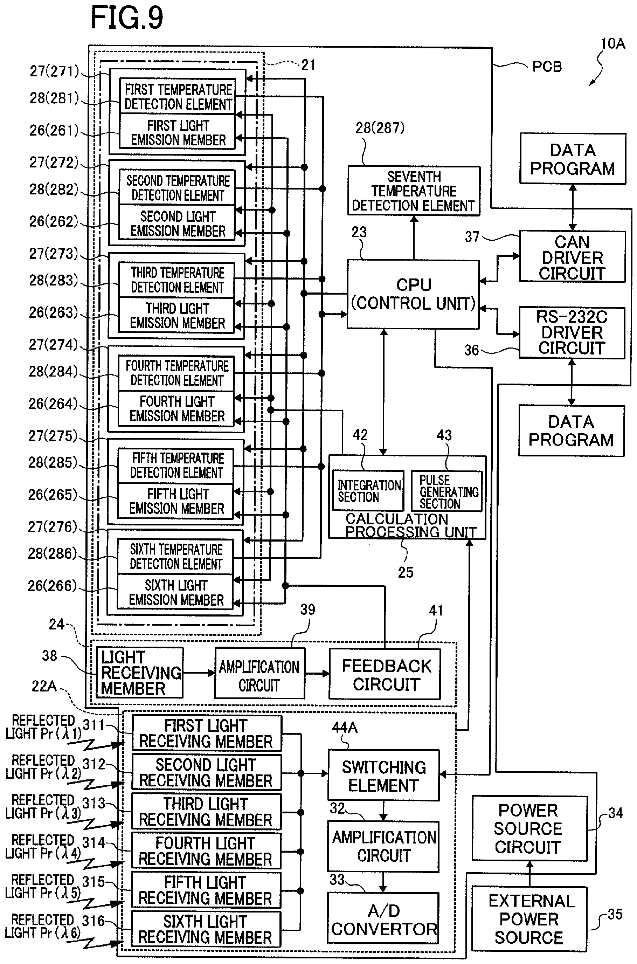

Next, a plant wavelength sensor device 10A is described as a plant wavelength sensor device of a Second Embodiment of the present invention with reference to Figs. 9 to 11. The plant wavelength sensor device 10A of the Second Embodiment differs from the plant wavelength sensor device 10 of the First Embodiment in how to emit the measurement light P having each wavelength range, the configuration of the light receiving unit 22A, and how to receive the reflected light Pr of the measurement light P having each wavelength range. As the plant wavelength sensor device 10A of the Second Embodiment is similar to that of the plant wavelength sensor device 10 of the First Embodiment, the same reference numbers are added to the similar configurations and detailed description thereof is omitted.

As shown in Fig. 9, the plant wavelength sensor device 10A of the Second Embodiment includes a light receiving unit 22A. The light receiving unit 22A includes six light receiving members 31, namely, a first light receiving member 311, a second light receiving member 312, a third light receiving member 313, a fourth light receiving member 314, a fifth light receiving member 315, and a sixth light receiving member 316. The first light receiving member 311 receives the reflected light Pr having the first wavelength range λ1. The first light receiving member 311 includes six PDs and a filter provided in front of the six PDs. The filter transmits a light having a wavelength range around 480 nm and does not transmit a light having the other wavelength ranges. The second light receiving member 312 receives the reflected light Pr having the second wavelength range λ2. The second light receiving member 312 includes six PDs and a filter provided in front of the six PDs. The filter transmits a light having a wavelength range around 700 nm and does not transmit a light having the other wavelength ranges. The third light receiving member 313 receives the reflected light Pr having the third wavelength range λ3. The third light receiving member 313 includes six PDs and a filter provided in front of the six PDs. The filter transmits a light having a wavelength range around 735 nm and does not transmit a light having the other wavelength ranges. The fourth light receiving member 314 receives the reflected light Pr having the fourth wavelength range λ4. The fourth light receiving member 314 includes six PDs and a filter provided in front of the six PDs. The filter transmits a light having a wavelength range around 808 nm and does not transmit a light having the other wavelength ranges. The fifth light receiving member 315 receives the reflected light Pr having the fifth wavelength range λ5. The fifth light receiving member 315 includes six PDs and a filter provided in front of the six PDs. The filter transmits a light having a wavelength range around 950 nm and does not transmit a light having the other wavelength ranges. The sixth light receiving member 316 receives the reflected light Pr having the sixth wavelength range λ6. The sixth light receiving member 316 includes six PDs and a filter provided in front of the six PDs. The filter transmits a light having a wavelength range around 1050 nm and does not transmit a light having the other wavelength ranges. The light receiving unit 22A receives the reflected light Pr in response to the input light receiving signal Sr similar to the light receiving unit 22 of the First Embodiment.

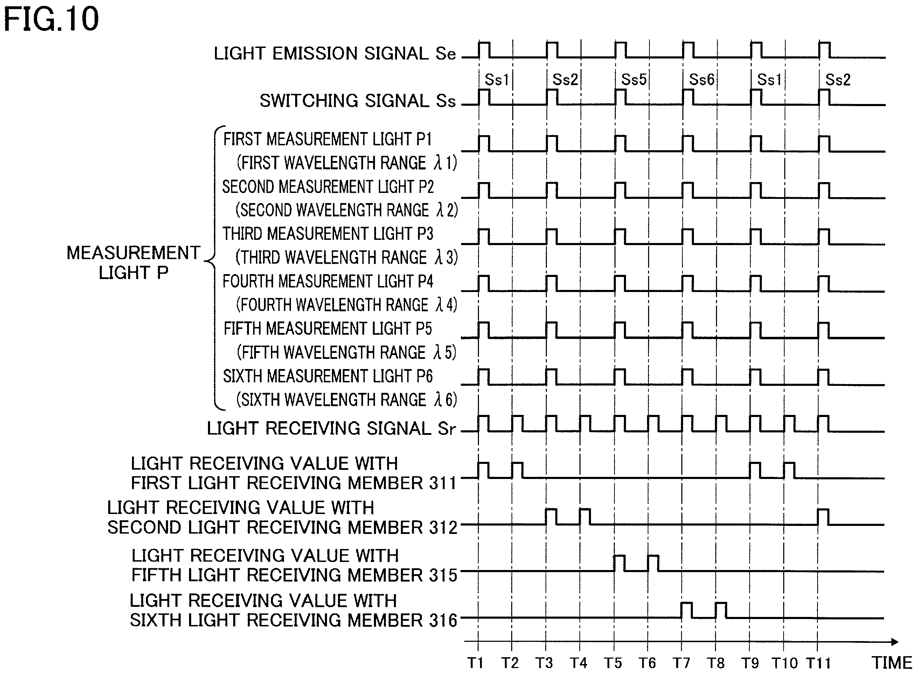

In the plant wavelength sensor device 10A, the light receiving unit 22A is provided with a switching element 44A instead of providing the switching element 44 between each light emission member 26 and the pulse generating section 43 (arithmetic processing unit 25). The switching element 44A selects any one of the light receiving members 31 to connect the selected light receiving member 31 to the amplification circuit 32. The switching element 44A connects the light receiving member 31, which corresponds to the switching signal Ss inputted from the CPU 23, to the amplification circuit 32. This switching signal Ss corresponding to the first light receiving member 311 is the first switching signal Ss1, the switching signal Ss corresponding to the second light receiving member 312 is the second switching signal Ss2, the switching signal Ss corresponding to the third light receiving member 313 is the third switching signal Ss3, the switching signal Ss corresponding to the fourth light receiving member 314 is the fourth switching signal Ss4, the switching signal Ss corresponding to the fifth light receiving member 315 is the fifth switching signal Ss5, and the switching signal Ss corresponding to the sixth light receiving member 316 is the sixth switching signal Ss6 (*see* Fig. 10). Accordingly, when the switching element 44A receives the n-th switching signal Ssn, the switching element 44A selects an electrical circuit to connect the n-th light receiving member 31n and the amplification circuit 32 to flow current in the selected electrical circuit and not to flow current in the other electrical circuits.

As a result, in the plant wavelength sensor device 10A, the light emission signal Se outputted from the pulse generating section 43 of the arithmetic processing unit 25 is directly inputted to each light emission member 26. Figs. 9, 10 show that the same light emission signal Se is inputted to each of the light emission members 26 from the pulse generating section 43. However, different light emission signals Se are outputted to the corresponding light emission members 26, respectively, as Se1 to Se6 of the First Embodiment. As the light emission signals Se are outputted to the respective light emission members 26, the light emission members 26 simultaneously emit light on a periodic basis (*see* Fig. 10). The switching signal Ss is outputted to the switching element 44A when the light emission signal Se is outputted (just before the light emission signal Se is outputted) to switch the electrical circuit of the switching element 44A. The light receiving member 31 thereby receives the reflected light Pr corresponding to a predetermined wavelength range. In the example of Fig. 10 showing an operation of estimating the amount of the protein (containing water), the first switching signal Ss1 is outputted to the switching element 44A to receive the reflected light Pr with the first light receiving member 311, the second switching signal Ss2 is outputted to the switching element 44A to receive the reflected light Pr with the second light receiving member 312, the fifth switching signal Ss5 is outputted to the switching element 44A to receive the reflected light Pr with the fifth light receiving member 315, and the sixth switching signal Ss6 is outputted to the switching element 44A to receive the reflected light with the sixth light receiving member 316.

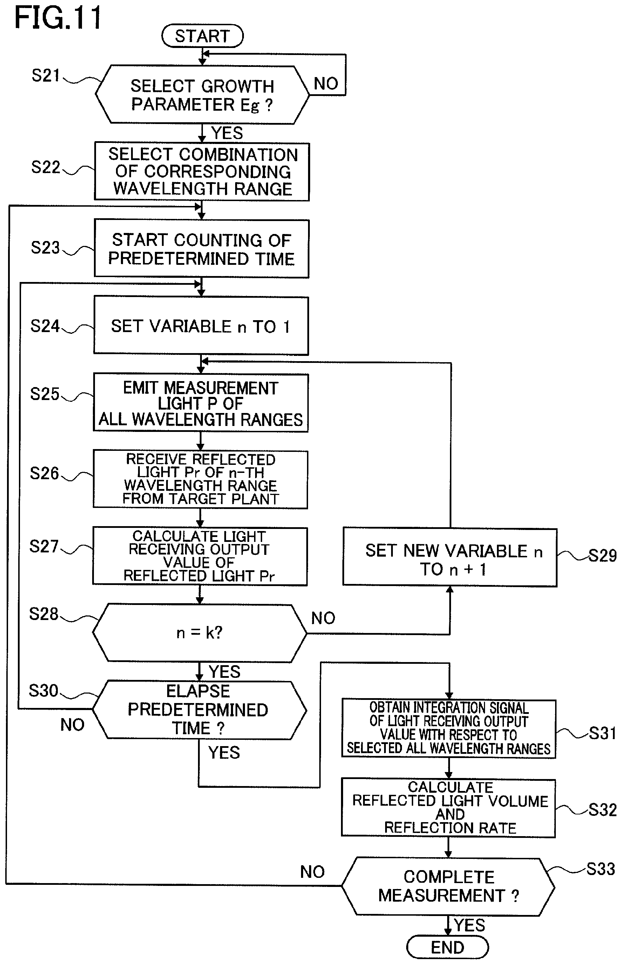

Next, a reflection measurement process of calculating a reflected light volume and a reflection rate of a target plant (crop Cr) with respect to a wavelength range for estimating the amount of the growth parameter Eg selected with the plant wavelength sensor device 10A is described with reference to Fig. 11. Fig. 11 is a flowchart showing the reflection measurement process which is executed by the control section (CPU 23) in the Second Embodiment. The control section (CPU 23) executes the reflection measurement process based on a program stored in the memory provided outside the control section (CPU 23) or the memory built in the control section (CPU 23). Hereinafter, each step of the flowchart of Fig. 11 is described. The flowchart of Fig. 11 starts in response to the turning on of the plant wavelength sensor device 10A.

In step S21, it is determined whether or not the growth parameter Eg is selected. When the determination result is YES, the process proceeds to step S22. When the determination result is NO, step S21 is repeated. Step S21 is similar to step S1 in the flowchart of Fig. 8.

In step S22, following the determination that the growth parameter Eg is selected in step S21, the combination of the selected growth parameter Eg and the corresponding wavelength range is selected. The process then proceeds to step S23. The step S22 is similar to step S2 in the flowchart of Fig. 8.

In step S23, following the selection of the combination in step S22 or the determination that the measurement has not been completed in step S33, the counting of the elapse time for integrating the light receiving values is started. The process then proceeds to step S24. The step S23 is similar to step S3 in the flowchart of Fig. 8.

In step S24, following the start of the counting of the elapse time in step S23 or the determination that a predetermined time has not been elapsed in step S30, the variable n is set to 1 (n = 1). The process then proceeds to step S25. The step S24 is similar to step S4 in the flowchart of Fig. 8.

In step S25, following the setting of the variable n to 1 in step S25 or the setting of the new variable n to n + 1, the measurement lights P of all of the wavelength ranges are emitted. The process then proceeds to step S26. In step S25, when all of the light emission members 26 receive the light emission signals Se, the light emission members 26 irradiate the growth plant (crop Cr) with the measurement lights P. Accordingly, the measurement lights P contain the lights of the set all wavelength ranges (in the Second Embodiment, the first to sixth wavelength ranges (λ1 to λ6)).

In step S26, following the emission of the measurement lights P of all wavelength ranges in step S25, the reflected light Pr of the n-th wavelength range of the measurement light P is received from the target plant (crop Cr). The process then proceeds to step S27. In step S26, the n-th switching signal Ssn is outputted to the switching element 44A to electrically connect the amplification circuit 32 and the light receiving member 31 corresponding to the n-th wavelength range in the light receiving unit 22A. In step S26, the light receiving member 31 corresponding to the n-th wavelength range receives the reflected light Pr in accordance with the light receiving signal Sr outputted in response to the light emission signal Se. In the Second Embodiment, similar to the First Embodiment, when the light emission member 26 corresponding to each wavelength range emits a light and when each of the light emission members 26 lights off just after the emission, the light receiving signal Sr is outputted. In step S26, the light receiving unit 22A obtains and outputs the light receiving value of the reflected light Pr corresponding to the n-th wavelength range when each light emission member 26 emits a light and the light receiving value of the reflected light Pr corresponding to the n-th wavelength range when each light emission member 26 lights off just after the emission.

In step S27, following the receiving of the reflected light Pr (light receiving value) of the n-th wavelength range in step S26, the light receiving output value of the received reflected light Pr (light receiving value) is calculated. The process then proceeds to step S28. The step S27 is similar to step S7 in the flowchart of Fig. 8.

In step S28, following the calculation of the light receiving output value in step S27, it is determined whether or not the variable n is equal to the number of wavelength ranges k (n = k ?). When the determination result is YES, the process proceeds to step S30. When the determination is NO, the process proceeds to step S29. The step S28 is similar to step S8 in the flowchart of Fig. 8.

In step S29, following the determination that the variable n in step S28 is not equal to the number of wavelength ranges k, the new variable n is set to n + 1. The process then returns to step S25. The step S29 is similar to step S9 in the flowchart of Fig. 8.

In step S30, following the determination that the variable n is equal to the number of wavelength ranges k, it is determined whether or not a predetermined time has elapsed. When the determination result is YES, the process proceeds to step S31. When the determination result is NO, the process returns to step S24. Step S30 is similar to step S10 in the flowchart of Fig. 8.

In step S31, following the determination that the predetermined time has elapsed, the integration signal of the light receiving output value with respect to the selected all wavelength ranges is obtained. The process then proceeds to step S32. Step S31 is similar to step S11 in the flowchart of Fig. 8.

In step S32, following the obtaining of the integration signal of the light receiving output value with respect to the selected all wavelength ranges in step S31, the reflected light volume and the reflection rate are calculated. The process then proceeds to step S33. Step S32 is similar to step S12 in the flowchart of Fig. 8.

In step S33, following the calculation of the reflected light volume and the reflection rate in step S32, it is determined whether or not the measurement with the plant wavelength sensor device 10A is completed. When the determination result is YES, the reflection measurement process is completed. When the determination result is NO, the process returns to step S23. Step S33 is similar to step S13 in the flowchart of Fig. 8.

Next, the operation of the plant wavelength sensor device 10A when estimating the amount of the growth parameter Eg is described with reference to Fig. 10 showing the example that the protein (containing water) is selected as the growth parameter Eg.

When the protein (containing water) is selected as the growth parameter Eg, the process proceeds from step S21 to step S22 in the flowchart of Fig. 11 to select the first wavelength range λ1 (480 nm), the second wavelength range λ2 (700 nm), the fifth wavelength range λ5 (950 nm), and the sixth wavelength range λ6 (1050 nm). After that, the process proceeds from step S23 to step S24 to step S25 in the flowchart of Fig. 11 to emit the measurement lights P (first measurement light P1 to sixth measurement light P6) having the recorded (set) all wavelength ranges (in the Second Embodiment, first to sixth wavelength ranges λ1 to λ6) from all of the light emission members 26 (*see* T1 of Fig. 10). The process then proceeds from step S26 to step S27 in the flowchart of Fig. 11 to receive the reflected light Pr from the target plant (crop Cr) with respect to the first wavelength range λ1 (480 nm) with the first light receiving member 311 and to calculate the light receiving output value (*see* T1, T2 of Fig. 10). After that, the process proceeds from step S28 to step S29 to step S25 to step S26 to step S27 in the flowchart of Fig. 11 to receive the reflected light Pr from the target plant (crop Cr) with respect to the second wavelength range λ2 (700 nm) with the second light receiving member 312 (*see* T3, T4 of Fig. 10), the reflected light Pr from the target plant (crop Cr) with respect to the fifth wavelength range λ5 (950 nm) with the fifth light receiving member 315 (*see* T5, T6 of Fig. 10), and the reflected light Pr from the target plant (crop Cr) with respect to the sixth wavelength range λ6 (1050 nm) with the sixth light receiving member 316 (*see* T7, T8 of Fig. 10), and to calculate the light receiving output values. Until the predetermined time has elapsed, the process repeats the above operations by proceeding from step S28 to S30 to S24 in the flowchart of Fig. 11. When the predetermined time has elapsed, the process proceeds from step S30 to step S31 to step S32 in the flowchart of Fig. 11 to calculate the reflected light receiving value (reflected light volume) representing the reflected light component from the target plant (crop Cr) and the reflection rate of the target plant (crop Cr) with respect to the four wavelength ranges. Accordingly, the amount of water and protein contained in the target plant (crop Cr) in the irradiation area IA can be estimated in the predetermined time. After that, until the exit operation, the process proceeds from step S32 to S33 to S23 in the flowchart of Fig. 11 to estimate the amount of water and protein contained in the target plant (crop Cr) in the next irradiation area IA.

When the nitrogen is selected as the growth parameter Eg, the third wavelength range λ3 (735 nm) and the fourth wavelength range λ4 (808 nm) are selected. The third light receiving member 313 receives the reflected light Pr from the target plant (crop Cr) with respect to the third wavelength range λ3 (735 nm), and the fourth light receiving member 314 receives the reflected light Pr from the target plant (crop Cr) with respect to the fourth light wavelength range λ4 (808 nm). The other operations similar to the above are also executed. Accordingly, when the growth parameter Eg is selected, the plant wavelength sensor device 10A can receive the reflected light volume and the reflection rate of the target plant (crop Cr) with respect to the measurement lights P having a plurality of wavelength ranges required for estimating the selected growth parameter Eg, and can estimate the amount of the growth parameter Eg.

As the plant wavelength sensor device 10A according to the Second Embodiment has the similar configuration as the plant wavelength sensor device 10 of the First Embodiment, the plant wavelength sensor device 10A according to the Second Embodiment can achieve the similar effect as that in the First Embodiment.

As the plant wavelength sensor device 10A of the Second Embodiment also emits the measurement light P from all of the light emission members 26, the plant wavelength sensor device 10A is simplified its control and/or configuration for emitting the measurement light P. As the plant wavelength sensor device 10A sequentially receives lights in chronological order with a plurality (n) of light receiving members 31, the plant wavelength sensor device 10A can easily and appropriately receive the reflected light Pr from the target plant (crop Cr) with respect to the measurement light P of each wavelength range.

As a result, the plant wavelength sensor device 10A of the Second Embodiment according to the present invention is applicable to various growth parameters Eg of the target plant (crop Cr).

In the Second Embodiment, although a plurality of light receiving members 31 is provided to correspond to the recorded (set) wavelength ranges, the configuration is not limited thereto. Any configuration can be used as long as the reflected light Pr of the recorded (set) wavelength range is received. For example, the configuration in which filters corresponding to the number of wavelength ranges are switchably provided in front of the single light receiving member 31 (six PDs) can be adopted.

Further, in the Second Embodiment, although a plurality of light receiving members 31 (six members in the Second Embodiment) is provided to correspond to the recorded (set) wavelength ranges, the configuration is not limited thereto. After all of the reflected lights Pr having wavelength ranges are received, the component of the reflected light Pr having the selected each wavelength range may be analyzed (extracted) and detected. With this configuration, the component of the reflected light Pr can be detected through the analysis (extraction) regardless of the combination of the wavelength and the growth parameter as long as the wavelength range is a recorded (set) wavelength range. Accordingly, the usability of the plant wavelength sensor device can be improved.

A plant wavelength sensor device of the present invention includes a light emission portion for emitting a measurement light to irradiate a target plant, a light receiving portion for receiving the measurement light reflected on the target plant as a reflected light; and a control section for controlling the light emission portion and the light receiving portion. The control section is configured to emit the measurement light having a wavelength range corresponding to a selected growth parameter from the light emission portion, to receive the reflected light from the target plant with the light receiving portion with respect to the measurement light having the wavelength range corresponding to the selected growth parameter, and to calculate a volume of the reflected light from the target plant as a reflected light volume with respect to the wavelength range corresponding to the growth parameter. With this configuration, the plant wavelength sensor device of the present invention is applicable to various growth parameters of a target plant

The control section is configured to record a selectable plurality of growth parameters and n wavelength ranges required for estimating all of the growth parameters, where n is a natural number equal to 3 or more, and the light emission member includes n light emission members for respectively emitting the measurement lights having the n wavelength ranges recorded in the control section. With this configuration, the target plant is easily irradiated with the measurement lights having a plurality of wavelength ranges required for estimating the selected growth parameter.

When the growth parameter is selected, the control section is configured to select each of the wavelength ranges corresponding to the selected parameter, to select each of the light emission members corresponding to each of the selected wavelength ranges, to emit each of the measurement lights from each of the selected light emission members, and to receive each reflected light with respect to each of the measurement lights with the light receiving portion. With this configuration, it becomes possible to irradiate the measurement lights having a plurality of wavelength ranges required for the selected growth parameter by operating the light emission member corresponding to the selected growth parameter from a preset plurality (n) of light emission members.

The control section is configured to emit each of the measurement lights in chronological order from each of the selected light emission members. With this configuration, the reflected light from the target plant can be easily and appropriately received with respect to the measurement light having each wavelength range.

The light receiving portion includes n light receiving members for respectively receiving the reflected lights of the n wavelength ranges recorded in the control section. When the growth parameter is selected, the control section extracts each of the wavelength ranges corresponding to the selected growth parameter, emits each of the measurement lights from each of the light emission members, selects each of the light receiving members corresponding to each of the extracted wavelength ranges, and to receive each of the reflected lights with each of the selected light receiving members. With this configuration, the irradiation of the measurement light can be easily controlled.

The control section is configured to receive each of the reflected lights corresponding to each of the selected light receiving members with each of the light receiving members in chronological order. With this configuration, the reflected light from the target plant can be easily received with respect to the measurement light having each wavelength range.

The control section is configured to record nitrogen as the selectable growth parameter, and to record a red light wavelength range and a near-infrared wavelength range as the wavelength ranges required for estimating the growth parameter. With this configuration, the amount of nitrogen as the growth parameter contained in the target plant can be easily and appropriately estimated.

The control section is configured to record protein as the selectable growth parameter, and to record two different visible wavelength ranges and a near-infrared wavelength range as the wavelength ranges required for estimating the growth parameter. With this configuration, the amount of protein as the growth parameter contained in the target plant can be easily and appropriately estimated.

The control section is configured to record water associated with estimation of the protein as the selectable growth parameter, and to record the near-infrared wavelength range as the wavelength range required for estimating the growth parameter. With this configuration, the amount of protein and the amount of water as the growth parameters contained in the target plant can be easily and appropriately estimated.

In the above embodiments, the plant wavelength sensor devices 10, 10A are described as an example of the plant wavelength sensor device according to the present invention. However, the plant wavelength sensor device of the present invention is not limited to thereto as long as a plant wavelength sensor device includes a light emission portion for emitting a measurement light to irradiate a target plant, a light receiving portion for receiving the measurement light reflected on the target plant as a reflected light, and a control section for controlling the light emission portion and the light receiving portion, wherein the control section is configured to emit the measurement light having a wavelength range corresponding to a selected growth parameter from the light emission portion, to receive the reflected light from the target plant with the light receiving portion with respect to the measurement light having the wavelength range corresponding to the selected growth parameter, and to calculate a volume of the reflected light from the target plant as a reflected light volume with respect to the wavelength range corresponding to the growth parameter, or a plant wavelength sensor device includes a light emission portion for emitting a measurement light to irradiate a target plant, a light receiving portion for receiving the measurement light reflected on the target plant as a reflected light, and a control section for controlling the light emission portion and the light receiving portion, wherein the control section is configured to emit the measurement light having a wavelength range corresponding to a selected growth parameter from the light emission portion, to receive the reflected light from the target plant with the light receiving portion with respect to the measurement light having the wavelength range corresponding to the selected growth parameter, and to calculate a reflection rate of the reflected light from the target plant and the measurement light with respect to the wavelength range corresponding to the growth parameter.

In each of the embodiments, although the nitrogen, the protein, and the water associated with the protein are recorded (set) as the growth parameters Eg, for example, only the two of them are recorded (set) as the growth parameters Eg. In addition to these or instead of these, phosphorus, potassium, chlorophyll, and carotenoid can be recorded (set) as the growth parameters Eg. The number of growth parameters Eg and/or the type of the growth parameter is not limited to those in the above embodiments. When the number and/or the type of growth parameter Eg are changed, the wavelength range applicable to the estimation of the growth parameter Eg is appropriately recorded (set).

In the above embodiments, the two wavelength ranges such as 735 nm and 808 nm are recorded (set) for estimating the nitrogen, the three wavelength ranges such as 480 nm, 700 nm, and 1050 nm are recorded (set) for estimating the protein, and one wavelength range such as 950 nm is recorded (set) for estimating the water associated with the estimation of the protein. However, the wavelength ranges with respect to the growth parameters Eg are not limited to these in the above embodiments. The wavelength range with respect to the growth parameter Eg is appropriately recorded (set) to be suitable for the estimation of the growth parameter Eg. Here, the nitrogen can be effectively estimated with the light (luminous flux) having the red light wavelength range and the light (luminous flux) having the infrared wavelength range, the protein can be effectively estimated with the lights (luminous flux) having the wavelength ranges as the two different visible lights and the light (luminous flux) having the near-infrared wavelength range, and the water associated with the estimation of the protein can be effectively estimated with the lights (luminous flux) having different near-infrared wavelength ranges.

In the above embodiments, the crop Cr as an agricultural crop is used as an example of the target plant. However, the target plant is not limited thereto. A plant may be used as the target plant as long as its growth status is estimated with the reflection rate of the measurement light.

In the above embodiments, the plant wavelength sensor devices 10, 10A are provided to the tractor TR equipped with the fertilizer spreader Fs. The fertilizer system is not limited to the above embodiments as long as the fertilizer system allows information to exchange between the fertilizer spreader and the plant wavelength sensor devices 10, 10A such that the fertilizer spreader Fs adjusts the spreading amount of the fertilizer based on the information of the amount of the growth parameter Eg and the information of the spectroscopy vegetation index (normalized difference vegetation index (NDVI)).

Although the plant wavelength sensor device according to the present invention has been described in terms of exemplary embodiments, it should not be limited thereto. It should be appreciated that variation or modification may be made in the embodiments by persons skilled in the art within the scope of the attached claims.

### CROSS-REFERENCE TO RELATED APPLICATION

The present application is based on and claims priority to Japanese Patent Application No. 2015-112242, filed on June 2, 2015.

## Claims

1. A plant wavelength sensor device (10A) for estimating a growth parameter (Eg), comprising:
a light emission unit (21) including at least three light emission members (261-266), each light emission member being configured for emitting measurement light (P) in a different wavelength range λn, where n is an index representing a natural number from 1 to at least 3, to irradiate a target plant (Cr);
a light receiving unit (22A) including for each wavelength range λn a corresponding light receiving member (311-316) for respectively detecting the measurement light (P) as reflected light (Pr) from the target plant (Cr);
a memory configured for storing a growth parameter (Eg), the wavelength ranges and the number thereof k required for estimating said growth parameter (Eg); and
a control unit (23,24,25) for controlling the light emission unit (21) and the light receiving unit (22A), wherein when the growth parameter (Eg) is selected the control unit (23,24,25) is configured to extract the combination of the wavelength ranges λ1 to λn and the corresponding number k thereof, required for estimating the selected growth parameter, from the data stored in the memory;
to control the light emission unit (21) to simultaneously emit measurement light (P) of all wavelength ranges λn on a periodic basis;
to control the light receiving unit (22) to detect the reflected light (Pr) from the target plant (Cr) only in the wavelength ranges corresponding to the predetermined wavelength ranges λ1 ... λn; and
to calculate either the intensities of the light reflected (Pr) from the target plant (Cr) as reflected light intensities with respect to the detected wavelength ranges corresponding to the growth parameter (Eg),
or the reflectivities of the target plant from the reflected and detected measurement light (P) with respect to the wavelength ranges corresponding to the growth parameter (Eg).

2. The plant wavelength sensor device according to claim 1, wherein the control unit (23,24,25) is configured to receive each of the reflected lights (Pr) corresponding to each of the selected light receiving members (311-316) with each of the light receiving members (31) in chronological order.

3. The plant wavelength sensor device according to any one of claims 1 or 2, wherein the control unit (23,24,25) is configured to record nitrogen as the selectable growth parameter (Eg), and to record a red light wavelength range and a near-infrared wavelength range as the wavelength ranges required for estimating the growth parameter (Eg).

4. The plant wavelength sensor device according to any one of claims 1 to 3, wherein the control unit (23,24,25) is configured to record protein as the selectable growth parameter (Eg), and to record two different visible wavelength ranges and a near-infrared wavelength range as the wavelength ranges required for estimating the growth parameter (Eg).

5. The plant wavelength sensor device according to claim 4, wherein the control unit (23,24,25) is configured to record water associated with estimation of the protein as the selectable growth parameter (Eg), and to record the near-infrared wavelength range as the wavelength range required for estimating the growth parameter (Eg).

## Patentansprüche

1. Pflanzenwellenlängensensorvorrichtung (10A) zum Schätzen eines Wachstumsparameters (Eg), umfassend:
eine Lichtemissionseinheit (21) mit mindestens drei Lichtemissionselementen (261-266), wobei jedes Lichtemissionselement eingerichtet ist, Messlicht (P) in einem anderen Wellenlängenbereich An zu emittieren, wobei n ein Index ist, der eine natürliche Zahl von 1 bis mindestens 3 darstellt, um eine Zielpflanze (Cr) zu bestrahlen;
eine Lichtempfangseinheit (22A), die für jeden Wellenlängenbereich An ein entsprechendes Lichtempfangselement (311-316) enthält, um jeweils das Messlicht (P) als reflektiertes Licht (Pr) von der Zielpflanze (Cr) zu erfassen;
einen Speicher, der eingerichtet ist, einen Wachstumsparameter (Eg), die Wellenlängenbereiche und deren Anzahl k, die für die Schätzung des Wachstumsparameters (Eg) erforderlich ist, zu speichern; und
eine Steuereinheit (23, 24, 25) zum Steuern der Lichtemissionseinheit (21) und der Lichtempfangseinheit (22A), wobei, wenn der Wachstumsparameter (Eg) ausgewählt ist, die Steuereinheit (23, 24, 25) eingerichtet ist, die Kombination der Wellenlängenbereiche λ1 bis An und die entsprechende Anzahl k davon, die für die Schätzung des ausgewählten Wachstumsparameters erforderlich ist, aus den im Speicher gespeicherten Daten zu extrahieren;
die Lichtemissionseinheit (21) zu steuern, um auf einer periodischen Basis gleichzeitig Messlicht (P) aller Wellenlängenbereiche An zu emittieren;
die Lichtempfangseinheit (22) zu steuern, um das von der Zielpflanze (Cr) reflektierte Licht (Pr) nur in den Wellenlängenbereichen zu erfassen, die den vorgegebenen Wellenlängenbereichen λ1...λn entsprechen; und
um entweder die Intensitäten des von der Zielpflanze (Cr) reflektierten Lichts (Pr) als reflektierte Lichtintensitäten in Bezug auf die erfassten Wellenlängenbereiche zu berechnen, die dem Wachstumsparameter (Eg) entsprechen,
oder die Reflektivitäten der Zielpflanze aus dem reflektierten und detektierten Messlicht (P) in Bezug auf die Wellenlängenbereiche, die dem Wachstumsparameter (Eg) entsprechen.

2. Pflanzenwellenlängensensorvorrichtung nach Anspruch 1, wobei die Steuereinheit (23, 24, 25) eingerichtet ist, jedes der reflektierten Lichter (Pr) entsprechend jedem der ausgewählten Lichtempfangselemente (311-316) mit jedem der Lichtempfangselemente (31) in chronologischer Reihenfolge zu empfangen.

3. Pflanzenwellenlängensensorvorrichtung nach einem der Ansprüche 1 oder 2, wobei die Steuerung (23, 24, 25) eingerichtet ist, Stickstoff als den auswählbaren Wachstumsparameter (Eg) aufzuzeichnen und einen Wellenlängenbereich für rotes Licht und einen Nahinfrarot-Wellenlängenbereich als die Wellenlängenbereiche zu aufzuzeichnen, die für die Schätzung des Wachstumsparameters (Eg) erforderlich sind.

4. Pflanzenwellenlängensensorvorrichtung nach einem der Ansprüche 1 bis 3, wobei die Steuereinheit (23, 24, 25) eingerichtet ist, Protein als auswählbaren Wachstumsparameter (Eg) aufzuzeichnen und zwei verschiedene sichtbare Wellenlängenbereiche und einen Nahinfrarot-Wellenlängenbereich als die Wellenlängenbereiche aufzuzeichnen, die für die Schätzung des Wachstumsparameters (Eg) erforderlich sind.

5. Pflanzenwellenlängensensorvorrichtung nach Anspruch 4, wobei die Steuereinheit (23, 24, 25) eingerichtet ist, dass Wasser, das mit der Schätzung des Proteins verbunden ist, als auswählbaren Wachstumsparameter (Eg) aufzuzeichnen und den Nahinfrarot-Wellenlängenbereich als den Wellenlängenbereich aufzeichnet, der für die Schätzung des Wachstumsparameters (Eg) erforderlich ist.

## Revendications

1. Dispositif de capteur de longueur(s) d'onde de plante (10A) pour estimer un paramètre de croissance (Eg), comprenant :
une unité d'émission de lumière (21) incluant au moins trois éléments d'émission de lumière (261-266), chaque élément d'émission de lumière étant configuré pour émettre une lumière de mesure (P) dans une plage de longueurs d'onde λn différente, où n est un index représentant un nombre naturel de 1 à au moins 3, pour irradier une plante cible (Cr) ;
une unité de réception de lumière (22A) incluant, pour chaque plage de longueurs d'onde λn, un élément de réception de lumière correspondant (311-316) pour respectivement détecter la lumière de mesure (P) en tant que lumière réfléchie (Pr) en provenance de la plante cible (Cr) ;
une mémoire configurée pour stocker un paramètre de croissance (Eg), les plages de longueurs d'onde et leur nombre k requis pour estimer ledit paramètre de croissance (Eg) ; et
une unité de commande (23, 24, 25) pour commander l'unité d'émission de lumière (21) et l'unité de réception de lumière (22A), dans lequel, lorsque le paramètre de croissance (Eg) est sélectionné, l'unité de commande (23, 24, 25) est configurée pour extraire la combinaison des plages de longueurs d'onde λ1 à λn et leur nombre k correspondant, requis pour estimer le paramètre de croissance sélectionné, à partir des données stockées dans la mémoire ;
pour commander l'unité d'émission de lumière (21) afin d'émettre simultanément une lumière de mesure (P) de toutes les plages de longueurs d'onde λn sur une base périodique ;
pour commander l'unité de réception de lumière (22) afin de détecter la lumière réfléchie (Pr) en provenance de la plante cible (Cr) seulement dans les plages de longueurs d'onde qui correspondent aux plages de longueurs d'onde prédéterminées λ1 ... λn ; et
pour calculer soit les intensités de la lumière réfléchie (Pr) en provenance de la plante cible (Cr) en tant qu'intensités de lumière réfléchie par rapport aux plages de longueurs d'onde détectées qui correspondent au paramètre de croissance (Eg),
soit les réflectivités de la plante cible à partir de la lumière de mesure réfléchie et détectée (P) par rapport aux plages de longueurs d'onde qui correspondent au paramètre de croissance (Eg).

2. Dispositif de capteur de longueur(s) d'onde de plante selon la revendication 1, dans lequel l'unité de commande (23, 24, 25) est configurée pour recevoir chacune des lumières réfléchies (Pr) correspondant à chacun des éléments de réception de lumière sélectionnés (311-316) à l'aide de chacun des éléments de réception de lumière (31) selon un ordre chronologique.

3. Dispositif de capteur de longueur(s) d'onde de plante selon l'une quelconque des revendications 1 ou 2, dans lequel l'unité de commande (23, 24, 25) est configurée pour enregistrer l'azote en tant que paramètre de croissance pouvant être sélectionné (Eg) et pour enregistrer une plage de longueurs d'onde de lumière rouge et une plage de longueurs d'onde des infrarouges proches en tant que plages de longueurs d'onde requises pour estimer le paramètre de croissance (Eg).

4. Dispositif de capteur de longueur(s) d'onde de plante selon l'une quelconque des revendications 1 à 3, dans lequel l'unité de commande (23, 24, 25) est configurée pour enregistrer une protéine en tant que paramètre de croissance pouvant être sélectionné (Eg) et pour enregistrer deux plages de longueurs d'ondes de la lumière visible différentes et une plage de longueurs d'onde des infrarouges proches en tant que plages de longueurs d'onde requises pour estimer le paramètre de croissance (Eg).

5. Dispositif de capteur de longueur(s) d'onde de plante selon la revendication 4, dans lequel l'unité de commande (23, 24, 25) est configurée pour enregistrer l'eau qui est associée à l'estimation de la protéine en tant que paramètre de croissance pouvant être sélectionné (Eg) et pour enregistrer la plage de longueurs d'onde des infrarouges proches en tant que plage de longueurs d'onde requise pour l'estimation du paramètre de croissance (Eg).
